# EUROPEAN PATENT SPECIFICATION

(11) **EP 2 121 616 B1**
(45) Date of publication and mention of the grant of the patent: **15.09.2010**
(21) Application number: 08709064.3
(22) Date of filing: 18.02.2008
(51) Int. Cl.: C07D 215/42

(54) **NOVEL 4-AMINO-QUINOLINE DERIVATIVES USEFUL AS ANTI-MALARIA DRUGS**
NEUARTIGE ALS ANTI-MALARIA-WIRKSTOFFE VERWENDBARE 4-AMINO-CHINOLINDERIVATE
NOUVEAUX DÉRIVÉS DE 4-AMINO-QUINOLINE À UTILISER EN TANT QU'ANTIPALUDÉENS

(30) Priority: 20.02.2007 DK 200700263; 21.02.2007 US 890862 P
(43) Date of publication of application: 25.11.2009
(73) Proprietor: Campiani, Giuseppe, 53042 Chianciano Terme (IT)
(72) Inventor: GEMMA, Sandra, I-73020 Martignano (IT); FATTORUSSO, Caterina, I-80128 Napoli (IT); KUKREJA, Gagan, I-53100 Siena (IT); JOSHI, Bhupendra, Prasad, I-53100 Siena (IT); BUTINI, Stefania, I-53100 Siena (IT); PERSICO, Marco, I-80067 Sorrento (IT); COCCONE, Salvator, Sanna, I-01010 Onano (IT); BERNETTI, Matteo, I-53045 Montepulciano (IT)
(74) Representative: Madsen, Inger Margrethe Schelde
(86) International application number: PCT/EP2008/051923
(87) International publication number: WO 2008/101891

(56) References cited:
- GEMMA ET AL: "Synthesis of N1-arylidene-N2-quinolyl- and N2-acrydinylhydrazones as potent antimalarial agents active against CQ-resistant P. falciparum strains" BIOORGANIC & MEDICINAL CHEMISTRY LETTERS, OXFORD, GB, vol. 16, no. 20, 15 October 2006 (2006-10-15), pages 5384-5388, XP005645275 ISSN: 0960-894X cited in the application

## Description

### TECHNICAL FIELD

The present invention relates to clotrimazole/quinoline hybrids useful as active ingredients of anti-malaria drugs. The compounds show a remarkable *in vitro* biological activity especially against the chloroquine-resistant *Plasmodium falciparum* strains and *in vivo* activity against *P. berghei.*

### BACKGROUND ART

Malaria, a disease caused by the parasite *Plasmodium falciparum* (*Pf*), represents one of the most widespread and deadly parasitic disease in man. For over 50 years, chloroquine (CQ) and related 4-aminoquinolines (amodiaquine, mefloquine, etc) have been the drugs of choice for malaria chemotherapy, until their efficacy was hampered by the spreading of CQ-resistant (CQ-R) *Pf* strains. Recently the amodiaquine regioisomer isoquine has been developed and is currently under investigation.

Although four species of the genus *Plasmodium* cause human malaria, most fatal malaria cases are due to infection by *Pf* which has a widespread geographic distribution and is most likely to be drug resistant. The parasites are transmitted through the bite of mosquitoes of the genus *Anopheles.* After infection of humans, *Pf* establishes an initial asymptomatic stage in liver cells followed by an erythrocytic stage which is responsible for the clinical symptoms of the disease. During the intra-erythrocytic stage of *Pf* life cycle, the catabolism of hemoglobin (Hb) is a key source of food for the parasite. Hb proteolysis occurs within the acidic (pH 4.9-5.4) digestive vacuole generating amino acids and releasing toxic free heme (ferriprotoporphyrin IX, FP). This latter is detoxified by the *Plasmodium* through polymerisation into an inert crystal known as the malaria pigment hemozoin. A second detoxification pathway is diffusion of free heme through the vacuolar membrane to the cytoplasm of the parasite and degradation by reduced glutathione.

The main mode of action of CQ is based on its ability to accumulate into the acidic *Pf* food vacuole (FV) and to interfere with the detoxification of free heme.

Studies have demonstrated that chlotrimazole (CLT), a potent anti-mycotic drug which act through inhibition of fungal P450 cytochrome 14α-lanosterol demethylase, shows moderate *in vitro* antimalarial activity against different CQ-resistant (CQ-R) and CQ-sensitive (CQ-S) strains of the malaria parasite *Pf*. The imidazole moiety of CLT behaves as an heme axial ligand which form stable, bulky and hydrophobic heme-CLT complex thereby enhancing the colloid-osmotic hemolysis and thus preventing heme from β-hematin formation, eventually disturbing the hemoglobin catabolism in the malarial parasite. In addition CLT seems to be involved in the inhibition of glutathione dependent heme degradation, resulting in enhancement of heme-induced hemolysis, thus damaging the cell membrane more than the free heme itself. Moreover, it has been suggested that in the presence of H₂O₂, CLT inhibits *Pf* hemoperoxidase. It is believed that this enzyme could be involved in the detoxification of H₂O₂.

Even though the *in vitro* antimalarial properties of CLT are interesting and it may represent a new hit in the field, our studies indicated that CLT is not active against *P. berghei in vivo* at doses up to 100 mg/Kg, making the use of CLT in antimalarial therapy not viable.

Gemma et al.; Bioorganic & Medicinal Chemistry Letters 2006 16 5384-5388, describe the synthesis of a series of *N*1-arylidene-*N*2-quinolyl- and *N*2-acrydinylhydrazones, useful as antimalarial agents. However, the 4-amino-quinoline derivatives of the present invention are not described.

Gemma et al.; Journal of Medicinal Chemistry 2007 50 595-598, describe a polycyclic pharmacophore related to clotrimazole, and its antimalarial activity. However, the 4-amino-quinoline derivatives of the present invention are not described.

### SUMMARY OF THE INVENTION

Starting from the interesting antimalarial properties of CLT, and taking also into account the mechanism of action of 4-aminoquinoline antimalarials like CQ, isoquine and amodiaquine, we designed and synthesized a series of CLT/quinoline hybrid compounds based on a novel pharmacophore with the aim to ameliorate the *in vitro* activity against CQ-S and CQ-R strains with respect to CLT and CQ, to overcome resistance to CQ and to obtain compounds active *in vivo* against the more common animal models of malaria.

The compounds of the invention present a remarkable *in vitro* biological activity especially against the CQ-R *Pf* strains, much higher than that of CLT and CQ, and a good antimalarial activity *in vivo* against *P. berghei* (ED₅₀ = 6.3 mg/Kg) which is promising for the development of new antimalarial drugs. Moreover, synthesis of the compounds of the invention is characterized by low costs of production which addresses to the economic burden associated with this orphan disease.

The compounds of the invention may be described as 4-amino-quinoline derivatives represented by Formula I, 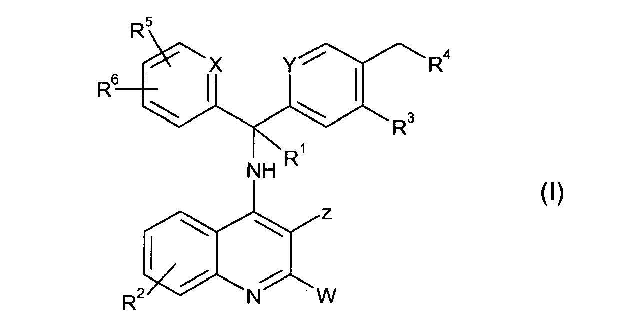 an isomer thereof or a mixture of its isomers, or a pharmaceutically acceptable salt thereof, wherein
X and Y both represent CH; or X and Y, together with the carbon atoms to which they are attached, form a bridge selected from C-C, C-CH₂-CH₂-C and C-CH=CH-C;
W and Z both represent hydrogen; or W and Z, together with the carbon atoms to which they are attached, form a benzo-fused ring -CH=CH-CH=CH-, which fused ring may optionally be substituted with halo;
R¹ represents hydrogen, phenyl, halo-substituted phenyl, 3,4-methylendioxyphenyl or (pyrrolidinylmethyl)phenyl;
R² represents halo, trifluoromethyl or alkoxy;
R³ represents hydrogen, halo, hydroxy, cyano, sulfonamido or dialkylsulfonamido;
R⁴ represents *N*,*N*-dialkyl-amino, pyrrolidinyl, piperazinyl, morpholinyl or imidazolyl;
R⁵ represents hydrogen, halo, cyano, hydroxy or -SO₂NH₂; and
R⁶ represents hydrogen, *N*,*N*-dialkyl-amino-methyl, pyrrolidinyl-methyl, piperazinyl-methyl, morpholinyl-methyl or 1*H*-imidazolyl-methyl;
as described in more details below.

In another aspect the invention provides pharmaceutical compositions comprising a therapeutically effective amount of a 4-amino-quinoline derivative of the invention, or a pharmaceutically-acceptable addition salt thereof, together with one or more adjuvants, excipients, carriers and/or diluents.

Viewed from another third aspect the invention relates to the use of the 4-amino-quinoline derivative of the invention, or a pharmaceutically-acceptable addition salt thereof, for the manufacture of a pharmaceutical composition/medicament.

Other objects of the invention will be apparent to the person skilled in the art from the following detailed description and examples.

### DETAILED DISCLOSURE OF THE INVENTION

In its first aspect the invention provides a 4-amino-quinoline derivative represented by Formula I, 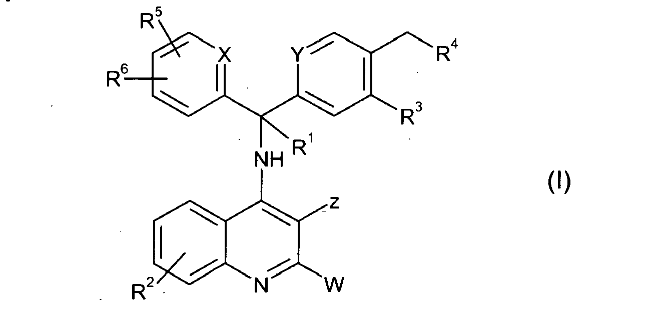 an isomer thereof or a mixture of its isomers, or a pharmaceutically acceptable salt thereof, wherein
X and Y both represent CH; or X and Y, together with the carbon atoms to which they are attached, form a bridge selected from C-C, C-CH₂-CH₂-C and C-CH=CH-C;
W and Z both represent hydrogen; or W and Z, together with the carbon atoms to which they are attached, form a benzo-fused ring -CH=CH-CH=CH-, which fused ring may optionally be substituted with halo;
R¹ represents hydrogen, phenyl, halo-substituted phenyl, 3,4-methylendioxyphenyl or (pyrrolidinylmethyl)phenyl;
R² represents halo, trifluoromethyl or alkoxy;
R³ represents hydrogen, halo, hydroxy, cyano, sulfonamido or dialkylsulfonamido;
R⁴ represents *N*,*N*-dialkyl-amino, pyrrolidinyl, piperazinyl, morpholinyl or imidazolyl;
R⁵ represents hydrogen, halo, cyano, hydroxy or -SO₂NH₂; and
R⁶ represents hydrogen, *N*,*N*-dialkyl-amino-methyl, pyrrolidinyl-methyl, piperazinyl-methyl, morpholinyl-methyl or 1*H*-imidazolyl-methyl.

In a preferred embodiment, however,
if R₂ represents halo, trifluoromethyl or alkoxy; X and Y both represent CH; and W and Z both represent hydrogen;
then the 4-amino-quinoline derivative of the invention is a compound of Formula I wherein
R₁ represents hydrogen, phenyl, fluorophenyl, 3,4-methylendioxyphenyl or (pyrrolidinylmethyl)phenyl;
R₃ represents hydrogen, halo or hydroxy;
R₄ represents *N*,*N*-dialkyl-amino, pyrrolidinyl, piperazynyl, morpholinyl or imidazolyl;
R₅ represents hydrogen, halo, cyano, hydroxy or SO₂NH₂; and
R₆ represents hydrogen, *N*,*N*-dialkyl-aminomethyl, pyrrolidinylmethyl, piperazynylmethyl, morpholinylmethyl or imidazolylmethyl.

In another preferred embodiment, however,
if R₂ represents alkoxy; and X and Y both represent CH;
then the 4-amino-quinoline derivative of the invention is a compound of Formula I wherein
R₁ represents hydrogen, phenyl, flurophenyl, 3,4-methylendioxyphenyl or (pyrrolidinylmethyl)phenyl;
R₃ represents hydrogen, halo or hydroxy;
R₄ represents *N*,*N*-dialkyl-amino, pyrrolidinyl, piperazynyl; morpholinyl or imidazolyl;
R₅ represents hydrogen, halo, cyano, hydroxy or SO₂NH₂;
R₆ represents hydrogen, *N*,*N*-dialkyl-aminomethyl, pyrrolidinylmethyl, piperazynylmethyl, morpholinylmethyl or imidazolylmethyl; and
W and Z, together with the carbon atoms to which they are attached, form a benzo-fused ring -CH=CH-CH=CH-, which fused ring may optionally be substituted with halo.

In a third preferred embodiment, however,
if R₂ represents halo, trifluoromethyl or alkoxy; X and Y, together with the carbon atoms to which they are attached, form a bridge selected from C-C, C-CH₂-CH₂-C and C-CH=CH-C; and R₁ represents hydrogen;
then the 4-amino-quinoline derivative of the invention is a compound of Formula I wherein W and Z both represent hydrogen; or
W and Z, together with the carbon atoms to which they are attached, form a benzo-fused ring -CH=CH-CH=CH-, which fused ring may optionally be substituted with halo;
R₃ represents hydrogen, halo or hydroxy;
R₄ represents *N*,*N*-dialkyl-amino, pyrrolidinyl, piperazynyl, morpholinyl or imidazolyl;
R₅ represents hydrogen, halo or hydroxy; and
R₆ represents hydrogen, *N*,*N*-dialkyl-aminomethyl, pyrrolidinylmethyl, piperazynylmethyl, morpholinylmethyl or imidazolylmethyl.

In a more preferred embodiment the 4-amino-quinoline derivative of the invention is a compound of Formula I, wherein
X and Y, together with the carbon atoms to which they are attached, form a bridge selected from C-C, C-CH₂-CH₂-C and C-CH=CH-C; and
W and Z both represent hydrogen; or
W and Z, together with the carbon atoms to which they are attached, form a benzo-fused ring -CH=CH-CH=CH-, which fused ring may optionally be substituted with halo;
R₁ represents hydrogen;
R₂ represents halo, trifluoromethyl or alkoxy;
R₃ represents hydrogen, halo or hydroxy;
R₄ represents *N*,*N*-dialkyl-amino, pyrrolidinyl, piperazynyl, morpholinyl or imidazolyl;
R₅ represents hydrogen, halo or hydroxy; and
R₆ represents hydrogen, *N*,*N*-dialkyl-aminomethyl, pyrrolidinylmethyl, piperazynylmethyl, morpholinylmethyl or imidazolylmethyl.

In a fourth preferred embodiment the 4-amino-quinoline derivative of the invention is a compound of Formula I, wherein X and Y both represent CH.

In a fifth preferred embodiment the 4-amino-quinoline derivative of the invention is a compound of Formula I, wherein X and Y, together with the carbon atoms to which they are attached, form a bridge selected from C-C, C-CH₂-CH₂-C and C-CH=CH-C.

In a more preferred embodiment X and Y, together with the carbon atoms to which they are attached, form a C-C bridge.

In another more preferred embodiment X and Y, together with the carbon atoms to which they are attached, form a C-CH₂-CH₂-C bridge.

In a third more preferred embodiment X and Y, together with the carbon atoms to which they are attached, form a C-CH=CH-C bridge.

In a sixth preferred embodiment the 4-amino-quinoline derivative of the invention is a compound of Formula I, wherein W and Z both represent hydrogen.

In a seventh preferred embodiment the 4-amino-quinoline derivative of the invention is a compound of Formula I, wherein W and Z, together with the carbon atoms to which they are attached, form a benzo-fused ring -CH=CH-CH=CH-, which fused ring may optionally be substituted with halo.

In an eighth preferred embodiment the 4-amino-quinoline derivative of the invention is a compound of Formula I, wherein W and Z, together with the carbon atoms to which they are attached, form a benzo-fused ring selected from -CH=CH-CH=CH- and -CH=CH-C(CI)=CH-.

In a more preferred embodiment W and Z, together with the carbon atoms to which they are attached, form a -CH=CH-C(CI)=CH- benzo-fused ring.

In a ninth preferred embodiment the 4-amino-quinoline derivative of the invention is a compound of Formula I, wherein
X and Y both represent CH;
W and Z both represent hydrogen;
R₁ represents hydrogen, phenyl, fluorophenyl, 3,4-methylendioxyphenyl or (pyrrolidinylmethyl)phenyl;
R₂ represents halo, trifluoromethyl or alkoxy;
R₃ represents hydrogen, halo, hydroxy, cyano, sulfonamido or dialkylsulfonamido;
R₄ represents *N*,*N*-dialkyl-amino, pyrrolidinyl, piperazynyl, morpholinyl or imidazolyl;
R₅ represents hydrogen, halo, cyano, hydroxy or SO₂NH₂; and
R₆ represents hydrogen, *N*,*N*-dialkyl-aminomethyl, pyrrolidinylmethyl, piperazynylmethyl, morpholinylmethyl or imidazolylmethyl.

In a more preferred embodiment
X and Y both represent CH;
W and Z both represent hydrogen;
R₁ represents hydrogen, phenyl, fluorophenyl, 3,4-methylendioxyphenyl or (pyrrolidinylmethyl)phenyl;
R₂ represents halo, trifluoromethyl or alkoxy;
R₃ represents hydrogen, halo or hydroxy;
R₄ represents *N*,*N*-dialkyl-amino, pyrrolidinyl, piperazynyl, morpholinyl or imidazolyl;
R₅ represents hydrogen, halo, cyano, hydroxy or SO₂NH₂; and
R₆ represents hydrogen, *N*,*N*-dialkyl-aminomethyl, pyrrolidinylmethyl, piperazynylmethyl, morpholinylmethyl or imidazolylmethyl.

In another more preferred embodiment R₁ represents hydrogen, phenyl, p-fluorophenyl, 3,4-methylendioxyphenyl or p-[(pyrrolidin-1-yl)methyl]phenyl.

In an even more preferred embodiment R₁ represents hydrogen, phenyl or *p-*fluorophenyl.

In a third more preferred embodiment R₂ represents 7-halo, in particular 7-chloro, 7-trifluoromethyl or 6-alkoxy, in particular 6-methoxy.

In a fourth more preferred embodiment R₃ represents hydrogen, chloro, fluoro or hydroxy.

In an even more preferred embodiment R₃ represents hydrogen.

In a fifth more preferred embodiment R₄ represents *N*,*N*-di-C₁₋₄-alkyl-amino, in particular *N*,*N*-dimethyl-amino or *N*,*N*-diethyl-amino, pyrrolidin-1-yl, piperazyn-1-yl, morpholin-4-yl or 1*H*-imidazol-1-yl.

In an even more preferred embodiment R₄ represents pyrrolidin-1-yl or morpholin-4-yl.

In a sixth more preferred embodiment R₅ represents hydrogen, fluoro, chloro, cyano, hydroxy or SO₂NH₂.

In an even more preferred embodiment R₅ represents halo, and in particular chloro.

In a seventh more preferred embodiment R₆ represents hydrogen, *N*,*N*-di-C₁₋₄-alkyl-aminomethyl, in particular *N*,*N*-dimethyl-aminomethyl or *N*,*N*-diethylaminomethyl, pyrrolidin-1-ylmethyl, piperazyn-1-ylmethyl, morpholin-4-ylmethyl or 1*H-*imidazol-1-ylmethyl.

In an even more preferred embodiment R₆ represents hydrogen.

In yet another more preferred embodiment
X and Y both represent CH;
W and Z both represent hydrogen;
R₁ represents hydrogen, phenyl or fluorophenyl;
R₂ represents halo, trifluoromethyl or alkoxy;
R₃ represents hydrogen;
R₄ represents pyrrolidinyl or morpholinyl;
R₅ represents halo; and
R₆ represents hydrogen.

In an eight preferred embodiment the 4-amino-quinoline derivative of the invention is a compound of Formula I, wherein
X and Y both represent CH;
W and Z, together with the carbon atoms to which they are attached, form a benzo-fused ring -CH=CH-CH=CH-, which fused ring may optionally be substituted with halo;
R₁ represents hydrogen, phenyl, flurophenyl, 3,4-methylendioxyphenyl or (pyrrolidinylmethyl)phenyl;
R₂ represents alkoxy;
R₃ represents hydrogen, halo, or hydroxy, cyano, sulfonamido or dialkylsulfonamido;
R₄ represents *N*,*N*-dialkyl-amino, pyrrolidinyl, piperazynyl, morpholinyl or imidazolyl;
R₅ represents hydrogen, halo, cyano, hydroxy or SO₂NH₂; and
R₆ represents hydrogen, *N*,*N*-dialkyl-aminomethyl, pyrrolidinylmethyl, piperazynylmethyl, morpholinylmethyl or imidazolylmethyl.

In a more preferred embodiment
X and Y both represent CH;
W and Z, together with the carbon atoms to which they are attached, form a benzo-fused ring -CH=CH-CH=CH-, which fused ring may optionally be substituted with halo;
R₁ represents hydrogen, phenyl, flurophenyl, 3,4-methylendioxyphenyl or (pyrrolidinylmethyl)phenyl;
R₂ represents alkoxy;
R₃ represents hydrogen, halo or hydroxy;
R₄ represents *N*,*N*-dialkyl-amino, pyrrolidinyl, piperazynyl, morpholinyl or imidazolyl;
R₅ represents hydrogen, halo, cyano, hydroxy or SO₂NH₂; and
R₆ represents hydrogen, *N*,*N*-dialkyl-aminomethyl, pyrrolidinylmethyl, piperazynylmethyl, morpholinylmethyl or imidazolylmethyl.

In another more preferred embodiment R₁ represents hydrogen, phenyl, p-flurophenyl, 3,4-methylendioxyphenyl or p-[(pyrrolidin-1-yl)methyl]phenyl.

In an even more preferred embodiment R₁ represents hydrogen.

In a third more preferred embodiment R₂ represents methoxy, and in particular 6-methoxy.

In a fourth more preferred embodiment R₃ represents hydrogen, chloro, fluoro or hydroxy.

In an even more preferred embodiment R₃ represents hydrogen.

In a fifth more preferred embodiment R₄ represents *N,N*-di-C₁₋₄-alkyl-amino, in particular *N*,*N*-dimethyl-amino or *N*,*N*-diethyl-amino, pyrrolidin-1-yl, piperazyn-1-yl, morpholin-4-yl or 1*H*-imidazol-1-yl.

In an even more preferred embodiment R₄ represents pyrrolidin-1-yl or morpholin-4-yl.

In a sixth more preferred embodiment R₅ represents hydrogen, fluoro, chloro, cyano, hydroxy or SO₂NH₂.

In an even more preferred embodiment R₅ represents halo, and in particular chloro.

In a seventh more preferred embodiment R₆ represents hydrogen, *N*,*N*-di-C₁₋₄-alkyl-aminomethyl, in particular *N*,*N*-dimethyl-aminomethyl or *N,N*-diethylaminomethyl, pyrrolidin-1-ylmethyl, piperazyn-1-ylmethyl, morpholin-4-ylmethyl or 1*H-*imidazol-1-ylmethyl.

In an even more preferred embodiment R₆ represents hydrogen.

In another more preferred embodiment
X and Y both represent CH;
W and Z, together with the carbon atoms to which they are attached, form a benzo-fused ring -CH=CH-CH=CH-, which fused ring may optionally be substituted with halo;
R₁ represents hydrogen, phenyl or flurophenyl;
R₂ represents alkoxy;
R₃ represents hydrogen;
R₄ represents pyrrolidinyl or morpholinyl;
R₅ represents halo; and
R₆ represents hydrogen.

In still another more preferred embodiment
X and Y both represent CH;
W and Z, together with the carbon atoms to which they are attached, form a benzo-fused ring -CH=CH-CH=CH-, which fused ring may optionally be substituted with halo;
R₁ represents hydrogen;
R₂ represents alkoxy;
R₃ represents hydrogen;
R₄ represents pyrrolidinyl or morpholinyl;
R₅ represents halo; and
R₆ represents hydrogen.

In an eight preferred embodiment the 4-amino-quinoline derivative of the invention is a compound of Formula I, wherein
X and Y, together with the carbon atoms to which they are attached, form a bridge selected from C-C, C-CH₂-CH₂-C and C-CH=CH-C; and
W and Z both represent hydrogen; or
W and Z, together with the carbon atoms to which they are attached, form a benzo-fused ring -CH=CH-CH=CH-, which fused ring may optionally be substituted with halo;
R₁ represents hydrogen;
R₂ represents halo, trifluoromethyl or alkoxy;
R₃ represents hydrogen, halo or hydroxy;
R₄ represents *N*,*N*-dialkyl-amino, pyrrolidinyl, piperazynyl, morpholinyl or imidazolyl;
R₅ represents hydrogen, halo or hydroxy; and
R₆ represents hydrogen, *N*,*N*-dialkyl-aminomethyl, pyrrolidinylmethyl, piperazynylmethyl, morpholinylmethyl or imidazolylmethyl.

In a more preferred embodiment R₂ represents halo, in particular 7-halo, trifluoromethyl in particular 7-trifluoromethyl or alkoxy, in particular methoxy.

In an even more preferred embodiment R₂ represents 7-halo, in particular 7-chloro, 7-trifluoromethyl or 6-alkoxy, in particular 6-methoxy.

In another more preferred embodiment R₃ represents hydrogen, fluoro, chloro or hydroxy.

In a third more preferred embodiment R₄ represents *N*,*N*-di-C₁₋₄-alkylamino, in particular *N*,*N*-dimethyl-amino or *N*,*N*-diethyl-amino, pyrrolidin-1-yl, piperazyn-1-yl, morpholin-4-yl or 1*H*-imidazol-1-yl.

In a fourth more preferred embodiment R₅ represents hydrogen, fluoro, chloro or hydroxy.

In a fifth more preferred embodiment R₆ represents hydrogen, *N*,*N*-di-C₁₋₄-alkyl-aminomethyl, in particular *N*,*N*-dimethyl-aminomethyl or *N*,*N*-diethylaminomethyl, pyrrolidin-1-ylmethyl, piperazyn-1-ylmethyl, morpholin-4-ylmethyl or 1*H-*imidazol-1-ylmethyl.

In a most preferred embodiment the 4-amino-quinoline derivative of the invention is
(±)-7-Chloro-*N*-{(3-chlorophenyl)[4-(pyrrolidin-1-ylmethyl)phenyl]methyl}-4-aminoquinoline (9a);
(±)-7-Chloro-*N*-{(3-chlorophenyl)[4-(morpholin-4-ylmethyl)phenyl]methyl}-4-aminoquinoline (9b);
(±)-7-Chloro-*N*-{[3-chloro-4-(pyrrolidin-1-ylmethyl)phenyl](3-chlorophenyl)methyl}-4-aminoquinoline (9c);
(±)-7-Chloro-*N*-{[3-chloro-4-(morpholin-4-ylmethyl)phenyl](3-chlorophenyl)methyl}-4-aminoquinoline (9d);
(±)-7-Chloro-*N*-{(4-chlorophenyl)[4-(pyrrolidin-1-ylmethyl)phenyl]methyl}-4-aminoquinoline (9e);
(±)-7-Chloro-*N*-{(4-chlorophenyl)[4-(morpholin-4-ylmethyl)phenyl]methyl}-4-aminoquinoline (9f);
(±)-7-Chloro-*N*-{(4-chlorophenyl)[4-(piperazin-1-ytmethyl)phenyl]methyl}-4-aminoquinoline (9g);
(±)-7-Chloro-*N*-{(4-chlorophenyl)[3-fluoro-4-(pyrrolidin-1-ylmethyl)phenyl]methyl}-4-aminoquinoline (9h);
(±)-*N*-{[3-Chloro-4-(pyrrolidin-1-ylmethyl)phenyl](3-chlorophenyl)methyl}-7-trifluoromethyl-4-aminoquinoline (9i);
(±)-*N*-{[3-Chloro-4-(morpholin-4-ylmethyl)phenyl](3-chlorophenyl)methyl}-7-trifluoromethyl-4-aminoquinoline (9j);
(±)-*N*-{(4-Chlorophenyl)[4-(pyrrolidin-1-ylmethyl)phenyl]methyl}-7-trifluoromethyl-4-aminoquinoline (9k);
(±)-*N*-{(4-Chlorophenyl)[3-fluoro-4-(pyrrolidin-1-ylmethyl)phenyl]methyl}-7-trifluoromethyl-4-aminoquinoline (91);
(±)-6-Methoxy-*N*-{(3-chlorophenyl)[4-(pyrrolidin-1-ylmethyl)phenyl]methyl}-4-aminoquinoline (9m);
(±)-*N*-{(3-Chlorophenyl)[4-(morpholin-4-ylmethyl)phenyl]methyl}-6-methoxy-4-aminoquinoline (9n);
(+)-*N*-{[3-Chloro-4-(pyrrolidin-1-ylmethyl)phenyl](3-chlorophenyl)methyl}-6-methoxy-4-aminoquinoline (90);
(±)-6-Methoxy-*N*-{(4-chlorophenyl)[4-(pyrrolidin-1-ylmethyl)phenyl]methyl}-4-aminoquinoline (9p); -
(±)-*N*-{(4-Chlorophenyl)[4-(morpholin-4-ylmethyl)phenyl]methyl}-6-methoxy-4-aminoquinoline (9q);
(±)-*N*-{(4-Chlorophenyl)[4-(piperazin-1-ylmethyl)phenyl]methyl}-6-methoxy-4-aminoquinoline (9r);
(±)-6-Chloro-*N*-{(4-chlorophenyl)[4-(pyrrolidin-1-ylmethyl)phenyl]methyl}-2-methoxy-9-aminoacridine (9s);
(±)-7-Chloro-*N*-1[4-(1*H*-imidazol-1-yl)methylphenyl](4-chlorophenyl)methyl}-4-aminoquinoline (9t);
(±)-7-Chloro-*N*-{(4-chlorophenyl)[4-(pyrrolidin-1-ylmethyl)phenyl]phenyl methyl}-4-aminoquinoline (15a);
(±)-7-Chloro-*N*-{(4-chlorophenyl)(4-fluorophenyl)[4-(pyrrolidin-1-ylmethyl)phenyl]methyl}-4-aminoquinoline (15b);
(±)-*N*-{(4-Chlorophenyl)[4-(pyrrolidin-1-ylmethyl)phenyl]phenylmethyl}-6-chloro-2-methoxy-9-aminoacridine (15c); or
*N*-{bis[3-Chloro-4-(pyrrolidin-1-ylmethyl)phenyl]phenylmethyl}-7-chloro-4-aminoquinoline (15d);
or a pharmaceutically acceptable addition salt thereof.

Any combination of two or more of the embodiments described herein is considered within the scope of the present invention.

### Definition of Substituents

In the context of this invention halo represents fluoro, chloro, bromo or iodo. Fluoro and chloro represent preferred halo atoms of the invention.

In the context of this invention an alkyl group designates a univalent saturated, straight or branched hydrocarbon chain. The hydrocarbon chain preferably contain of from one to eighteen carbon atoms (C₁₋₁₈-alkyl), more preferred of from one to six carbon atoms (C₁₋₆-alkyl; lower alkyl), including pentyl, isopentyl, neopentyl, tertiary pentyl, hexyl and isohexyl. In a preferred embodiment alkyl represents a C₁₋₄-alkyl group, including butyl, isobutyl, secondary butyl and tertiary butyl. In another preferred embodiment of this invention alkyl represents a C₁₋₃-alkyl group, which may in particular be methyl, ethyl, propyl or isopropyl.

In the context of this invention an alkoxy group designates an "alkyl-O-" group, wherein alkyl is as defined above. Examples of preferred alkoxy groups of the invention include methoxy and ethoxy.

### Pharmaceutically Acceptable Salts

The 4-amino-quinoline derivatives of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Examples of pharmaceutically acceptable cationic salts of a chemical compound of the invention include, without limitation, the sodium, the potassium, the calcium, the magnesium, the zinc, the aluminium, the lithium, the choline, the lysine, and the ammonium salt, and the like, of a chemical compound of the invention containing an anionic group. Such cationic salts may be formed by procedures well known and described in the art.

### Isomers

It will be appreciated by those skilled in the art that the 4-amino-quinoline derivatives of the present invention may exist in different stereoisomeric forms, including enantiomers, diastereomers, as well as geometric isomers (cis-trans isomers). The invention includes all such isomers and any mixtures thereof including racemic mixtures.

Methods for the resolving the optical isomers are known in the art. Such methods include those described by Jaques J, Collet A, & Wilen S in "Enantiomers, Racemates and Resolutions", John Wiley and Sons, New York (1981).

Optical active compounds can also be prepared from optically active starting materials or intermediates.

### Methods of Preparation

The 4-amino-quinoline derivatives of the invention may be prepared by conventional methods for chemical synthesis, e.g. those described in the working examples. The starting materials for the processes described in the present application are known or may readily be prepared by conventional methods from commercially available chemicals.

The 4-amino-quinoline derivatives may be prepared from readily available starting materials by following general methods and procedures. It will be appreciated that where typical or preferred experimental conditions (i.e. reaction temperatures, time, moles of reagents, solvents, etc.) are given, other experimental conditions can also be used, unless otherwise stated. Optimum reaction conditions may vary with the particular reactants or solvents used, but such conditions can be determined by one skilled in the art by routine optimization procedures. Specific reference is made to the methods described in the Examples and to the Schemes 1-2.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The compounds of the invention present a remarkable *in vitro* biological activity especially against the CQ-R Pf strains, much higher than that of CLT and CQ, and a good antimalarial activity *in vivo* against *P. berghei* which is promising for the development of new antimalarial drugs.

Preliminary evaluation of the putative mechanism of action of the compounds showed that interference with heme polymerisation and detoxification processes could be involved as *in vitro* inhibition of β-hematin formation was observed. Moreover, in contrast to CLT, the compounds of the invention do not interact with heme in P450 cytochromes as demonstrated by their lack of anti-fungal activity being selective for free heme. Free heme represents a valuable target in the design of new antimalarial agents as the lack of interaction with a specific protein target can decrease the potential of inducing resistance under drug pressure.

Therefore, in another aspect the invention relates to use of the 4-aminoquinoline derivatives of the invention, or a pharmaceutically-acceptable addition salt thereof, or a prodrug thereof, for the manufacture of a pharmaceutical composition/medicament for the treatment, prevention or alleviation of an infectious disease, disorder or condition of a mammal, including a human, which disease, disorder or condition is caused by a parasite of the genus *Plasmodium.*

In a more preferred embodiment the disease, disorder or condition is caused by *P. falciparum, P. berghei, P. vivax, P. ovale, P. malaria* or *P. knowlesi.*

In a most preferred embodiment invention relates to use of the 4-aminoquinoline derivatives of the invention for the treatment, prevention or alleviation of malaria. Such use includes the treatment of a mammal suffering from malaria infection or at risk of being infected.

The term "therapeutically effective amount" as used herein refers to an amount of a therapeutic agent needed to treat, ameliorate a targeted disease or condition, or to exhibit a detectable therapeutic effect.

For any compound, the therapeutically effective dose can be estimated initially either in cell culture assays, for example, *P. falciparum* strains, or in animal models, usually mice, rats or monkeys.

The animal model may also be used to determine the appropriate concentration range and route of administration. Such information can then be used to determine useful doses and routes for administration in humans.

The precise effective amount for a human subject will depend upon the severity of the disease state, general health of the subject, age, weight and gender of the subject, diet, time and frequency of administration, drug combination (s), reaction sensitivities and tolerance/response to therapy. This amount can be determined by routine experimentation and is within the judgement of the clinician. Generally, an effective dose will be from 0.01 mg/Kg to 100 mg/Kg, preferably 0.05 mg/Kg to 50 mg/Kg. Compositions may be administered individually to a patient or may be administered in combination with other agents, drugs.

### Pharmaceutical Compositions

In another aspect the invention provides novel pharmaceutical compositions comprising a therapeutically effective amount of the 4-amino-quinoline derivative of the invention.

The pharmaceutical composition of the invention comprising the 4-aminoquinoline derivative of the invention may further comprise known active principles such as artemisins (DHA, artesunate, etc.).

While a chemical compound of the invention for use in therapy may be administered in the form of the raw chemical compound, it is preferred to introduce the active ingredient, optionally in the form of a physiologically acceptable salt, or in the form of a prodrug, in a pharmaceutical composition together with one or more adjuvants, excipients, carriers, buffers, diluents and/or other customary pharmaceutical auxiliaries.

In a preferred embodiment, the invention provides pharmaceutical compositions comprising the 4-amino-quinoline derivative of the invention, or a pharmaceutically acceptable salt or derivative thereof, together with one or more pharmaceutically acceptable carriers, and, optionally, other therapeutic and/or prophylactic ingredients, know and used in the art. The carrier(s) must be "acceptable" in the sense of being compatible with the other ingredients of the formulation and not harmful to the recipient thereof.

Such carriers include antibodies and other polypeptides, genes and other therapeutic agents such as liposomes, provided that the carrier does not itself induce the production of antibodies harmful to the individual receiving the composition, and which may be administered without undue toxicity.

Suitable carriers may be large, slowly metabolised macromolecules such as proteins, polysaccharides, polylactic acids, polyglycolic acids, polymeric amino acids, amino acid copolymers and inactive virus particles.

Pharmaceutically acceptable carriers in therapeutic compositions may additionally contain liquids such as water, saline, glycerol and ethanol. Additionally, auxiliary substances, such as wetting or emulsifying agents, pH buffering substances, and the like, may be present in such compositions. Such carriers enable the pharmaceutical compositions to be formulated as tablets, pills, dragees, capsules, liquids, gels, syrups, slurries, suspensions, and the like, for ingestion by the patient.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be prepared by any person skilled in the art, by use of standard methods and conventional techniques, appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/Kg i.v. and 100 mg/Kg p.o. Preferred ranges are from about 0.1 µg/kg to about 10 mg/Kg/day i.v., and from about 1 µg/kg to about 100 mg/Kg/day p.o.

### Methods of Therapy

In another aspect the invention provides compounds for use in the treatment, prevention or alleviation of an infectious disease, disorder or condition of a living animal body, including a human, which disorder, disease or condition is caused by a parasite of the genus *Plasmodium,* which comprises the step of administering to such a living animal body in need thereof, a therapeutically effective amount of the 4-aminoquinoline derivative of the invention, or a pharmaceutically-acceptable addition salt thereof .

In the context of this invention the term "treatment" covers treatment, prevention, prophylaxis or alleviation, and the term "disease" covers illnesses, diseases, disorders and conditions related to the disease in question.

The preferred indications contemplated according to the invention are those stated above.

It is at present contemplated that a suitable dosage of the active pharmaceutical ingredient (API) is within the range of from about 0.1 to about 1000 mg API per day, more preferred of from about 10 to about 500 mg API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

### EXAMPLES

The invention is further illustrated with reference to the following examples, which are not intended to be in any way limiting to the scope of the invention as claimed.

### Example 1

### Synthesis of Derivatives 9a-s according to Scheme 1

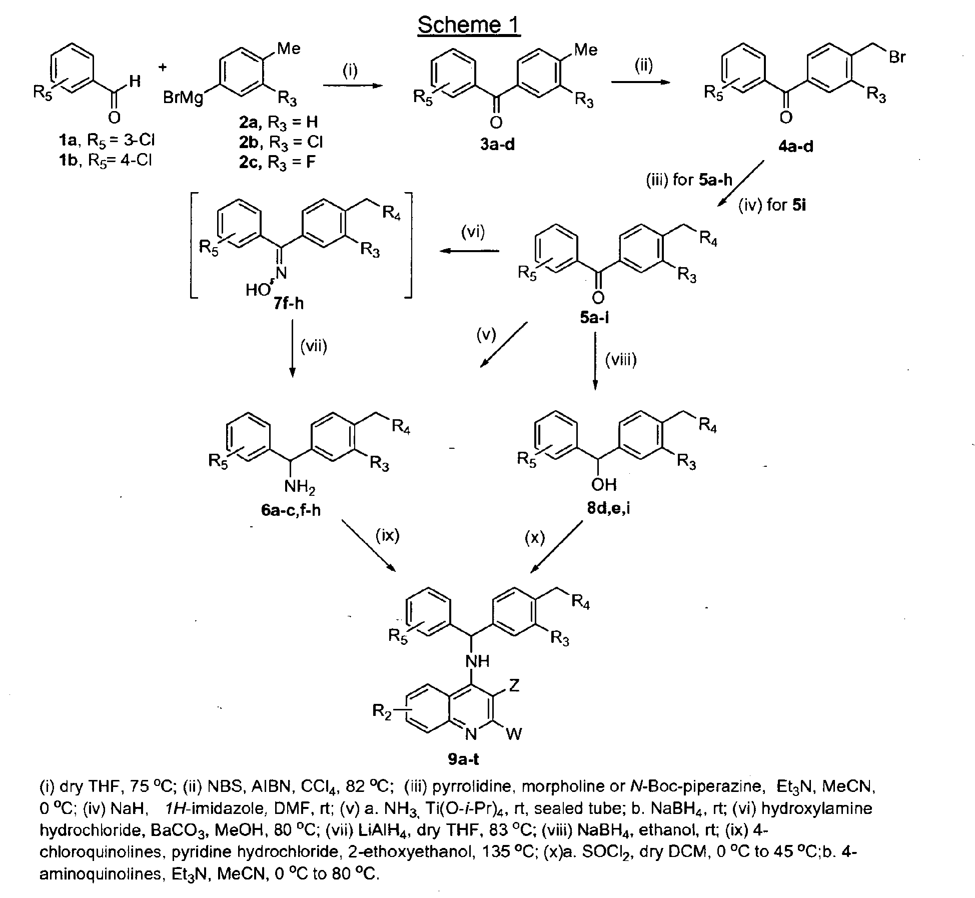

**Table 1**

| | **R₂** | **R₃** | **R₄** | **R₅** | **Z** | **W** |
|---|---|---|---|---|---|---|
| **Compounds 3,4** | | | | | | |
| **a** | - | H | - | 3-Cl | - | - |
| **b** | - | H | - | 4-Cl | - | - |
| **c** | - | Cl | - | 3-Cl | - | - |
| **d** | - | F | - | 4-Cl | - | - |
| **Compounds 5-8** | | | | | | |
| **a** | - | H | Pyrrolidin-1-yl | 3-Cl | - | - |
| **b** | - | H | Pyrrolidin-1-yl | 4-Cl | - | - |
| **c** | - | H | Morpholin-4-yl | 3-Cl | - | - |
| **d** | - | H | Morpholin-4-yl | 4-Cl | - | - |
| **e** | - | H | *N*-Boc-piperazin-2-yl | 4-Cl | - | - |
| **f** | - | Cl | Pyrrolidin-1-yl | 3-Cl | - | - |
| **g** | - | Cl | Morpholin-4-yl | 3-Cl | - | - |
| **h** | - | F | Pyrrolidin-1-yl | 4-Cl | - | - |
| **i** | - | H | 1*H*-Imidazol-1-yl | 4-Cl | - | - |
| **Compounds 9** | | | | | | |
| **9a** | 7-Cl | H | Pyrrolidin-1-yl | 3-Cl | H | H |
| **9b** | 7-Cl | H | Morpholin-4-yl | 3-Cl | H | H |
| **9c** | 7-Cl | Cl | Pyrrolidin-1-yl | 3-Cl | H | H |
| **9d** | 7-Cl | Cl | Morpholin-4-yl | 3-Cl | H | H |
| **9e** | 7-Cl | H | Pyrrolidin-1-yl | 4-Cl | H | H |
| **9f** | 7-Cl | H | Morpholin-4-yl | 4-Cl | H | H |
| **9g** | 7-Cl | H | Piperazin-2-yl | 4-Cl | H | H |
| **9h** | 7-Cl | F | Pyrrolidin-1-yl | 4-Cl | H | H |
| **9i** | 7-CF₃ | Cl | Pyrrolidin-1-yl | 3-Cl | H | H |
| **9j** | 7-CF₃ | Cl | Morpholin-4-yl | 3-Cl | H | H |
| **9k** | 7-CF₃ | H | Pyrrolidin-1-yl | 4-Cl | H | H |
| **9l** | 7-CF₃ | F | Pyrrolidin-1-yl | 4-Cl | H | H |
| **9m** | 6-OMe | H | Pyrrolidin-1-yl | 3-Cl | H | H |
| **9n** | 6-OMe | H | Morpholin-4-yl | 3-Cl | H | H |
| **9o** | 6-OMe | Cl | Pyrrolidin-1-yl | 3-Cl | H | H |
| **9p** | 6-OMe | H | Pyrrolidin-1-yl | 4-Cl | H | H |
| **9q** | 6-OMe | H | Morpholin-4-yl | 4-Cl | H | H |
| **9r** | 6-OMe | H | Piperazin-2-yl | 4-Cl | H | H |
| **9s** | 6-OMe | H | Pyrrolidin-1-yl | 4-Cl | -CH=CH- C(Cl)=C- | |
| **9t** | 7-Cl | H | 1*H*-Imidazol-1-yl | 4-Cl | H I | H |

### (3-Chlorophenyl)(4-methylphenyl)methanone (3a)

To a stirred solution of magnesium turnings (1.13 g, 46.77 mmol) in anhydrous tetrahydrofuran (40 mL), a solution containing 4-bromotoluene **2** (8.0 g, 46.77 mmol) in dry THF (40 mL) was slowly added. The reaction mixture was stirred and heated under reflux until the reaction started, when the source of heat was removed until the exothermic reaction ceased. Heating was then continued under reflux for 1 h, when nearly all the magnesium had been consumed. Thereafter, the reaction was cooled to 0°C and a solution of 3-chlorobenzaldehyde **(1 a,** 10.0 mL, 93.54 mmol) in 70 mL of anhydrous tetrahydrofuran was added drop wise and the resulting solution was heated at 75°C for 4h. The reaction mixture was quenched with 50 mL of 20% of ammonium chloride solution. The aqueous layer was extracted with ethyl acetate (3 × 40 mL) and the combined organic layers were washed with brine, dried over Na₂SO₄ and evaporated under reduced pressure. The crude residue was purified by flash chromatography (2% ethyl acetate in hexane) to give **6a** as a white solid (8.78 g, 70%); ¹H NMR (200 MHz, CDCl₃) δ 7.74-7.67 (m, 3H), 7.65-7.51 (m, 1H), 7.43-7.39 (m, 1H), 7.35-7.26 (m, 3H), 2.43 (s, 3H).

### (4-Chlorophenyl)(4-methylphenyl)methanone (3b)

Starting from **1b** (5.0 g, 29.23 mmol) and **2,** the title compound was prepared following the above described procedure and was obtained as a white solid (5.37 g, 80%); ¹H NMR (300 MHz, CDCl₃) δ 7.75-7.67 (m, 4H), 7.46-7.43 (m, 2H), 7.30-7.26 (m, 2H), 2.44 (s, 3H).

### (3-Chloro-4-methylphenyl)(3-chlorophenyl)methanone (3c)

Starting from **1a** and **2b** (15 g, 73 mmol), the title compound was prepared following the procedure described for **3a** and after flash chromatography (ethyl acetate/*n*-hexane 40:1) was obtained as a white solid (18.0 g, 78%); ¹H NMR (CDCl₃) δ 7.78-7.75 (m, 2H), 7.64-7.55 (m, 3H), 7.45-7.44 (m, 2H), 2.46 (s, 3H).

### (4-Chlorophenyl)(3-fluoro-4-methylphenyl)methanone (3d)

Starting from **1b** and **2c** (10 g, 53 mmol), the title compound was prepared following the procedure described for **3a** and after flash chromatography (ethyl acetate/*n*-hexane 1:30) was obtained as a white solid (11.2 g, 85%); ¹H NMR (300 MHz, CDCl₃) δ 7.71 (d, 2H, *J* = 8.5 Hz), 7.47-7.44 (m, 4H), 7.32-7.26 (m, 1H), 2.36 (s, 3H); ESI MS *m*/*z* 250 (M+H)⁺.

### [4-(Bromomethyl)phenyl](3-chlorophenyl)methanone (4a)

To a solution of compound **3a** (3.1 g, 13.5 mmol) in CCl₄ (40 mL) was added NBS (2.8 g, 15.6 mmol) and AIBN (catalytic amount). The solution was heated under reflux for 3h. After cooling to room temperature, the succinimide was filtered off and the solvent was evaporated. The crude residue was purified by flash chromatography (5% ethyl acetate in hexane) to afford **4a** as a white solid (3.4 g, 81%); ¹H NMR (200 MHz, CDCl₃) δ 7.74-7.63 (m, 4H), 7.59-7.38 (m, 4H), 4.49 (s, 2H).

### [4-(Bromomethyl)phenyl](4-chlorophenyl)methanone (4b)

Starting from **3b** (3.6 g, 15.5 mmol), the title compound was prepared following the procedure described for **4a** and was obtained as a white solid (3.9 g, 81%); δ ¹H NMR (300 MHz, CDCl₃) δ 7.76-7.72 (m, 4H), 7.52-7.49 (m, 4H), 4.52 (s, 2H).

### [4-(Bromomethyl)-3-chlorophenyl](3-chlorophenyl)methanone (4c)

Starting from **3b** (14 g, 53.7 mmol), the title compound was prepared following the procedure described for **4a** and after flash chromatography (ethyl acetate/*n*-hexane 1:40) was obtained as a white solid (11.5 g, 62%); ¹H NMR (CDCl₃) δ 7.82-7.77 (m, 2H), 7.66-7.59 (m, 4H), 7.47-7.42 (m, 2H), 4.63 (s, 2H).

### [4-(bromomethyl)-3-fluorophenyl](4-chlorophenyl)methanone (4d)

Starting from **3d** (5 g, 20.2 mmol), the title compound was prepared following the procedure described for **4a** and after flash chromatography (ethyl acetate/n-hexane 1:30) was obtained as a white solid (5 g, 76%); ¹H NMR (300 MHz, CDCl₃) δ 7.75 (d, 2H, *J* = 8.5 Hz),7.54-7.47 (m, 5H), 4.55 (s, 2H); ESI MS *m*/*z* 329 (M+H)⁺.

### (3-Chlorophenyl)[4-(pyrrolidin-1-ylmethyl)phenyl]methanone (5a)

To a stirred solution of **4a** (3.5 g, 11.30 mmol) in dry acetonitrile (75 mL) was added pyrrolidine (1.41 mL, 16.96 mmol) and triethylamine (3.14 mL, 22.61 mmol) at 0°C and the resulting mixture was allowed to stir for 1h at 0°C. Thereafter, the reaction was quenched with 2 mL of water and the solvent was evaporated under reduced pressure. The residue was treated with water and extracted with chloroform (2 × 40 mL). The combined organic layers were washed with brine, dried over sodium sulphate and evaporated. The residue was chromatographed (2% methanol in dichloromethane) to afford **5a** as a yellow viscous oil (2.50 g, 73%); ¹H NMR (200 MHz, CDCl₃) δ 7.70-7.56 (m, 3H), 7.49-7.13 (m, 5H), 3.62 (s, 2H), 2.47 (m, 4H), 1.73 (m, 4H); ESI MS *m*/*z* 300(M+H)⁺.

### (4-Chlorophenyl)[4-(pyrrolidin-1-ylmethyl)phenyl]methanone (5b)

Starting from **4b** (3.0 g, 9.69 mmol), the title compound was prepared following the above described procedure and was obtained as a yellow viscous oil (2.16 g, 74%); ¹H NMR (300 MHz, CDCl₃) δ 7.76-7.68 (m, 4H), 7.48-7.39 (m, 4H), 3.68 (s, 2H), 2.52 (m, 4H), 1.77 (m, 4H); ESI MS *m*/*z* 300 (M+H)⁺.

### (3-Chlorophenyl)[4-(morpholin-4-ylmethyl)phenyl]methanone (5c)

Starting from **4a** and morpholine, the title compound was prepared following the above described procedure and was obtained as a yellow viscous oil; ¹H NMR (300 MHz, CDCl₃) δ 7.76-7.73 (m, 3H), 7.67-7.63 (m, 1H), 7.56-7.53 (m, 1H), 7.48-7.44 (m, 2H), 7.41-7.38 (m, 1H), 3.74-3.71 (m, 4H), 3.57 (s, 2H), 2.49-2.47 (m, 4H).

### (4-Chlorophenyl)[4-(morpholin-4-ylmethyl)phenyl]methanone (5d)

Starting from **4b** (0.58 g, 1.84 mmol), the title compound was prepared following the above described procedure and was obtained as a yellow oil (0.51 g, 87%); ¹H NMR (300 MHz, CDCl₃) δ 7.25-7.23 (m, 8H), 5.67 (s, 1H), 4.03 (bs, 1H), 3.57 (m, 4H), 3.41 (s, 2H), 3.38 (m, 4H); ESI MS *m*/*z* 318 (M+H)⁺.

### (4-Chlorophenyl){4-[4-(tert-butoxycarbonyl)piperazin-1-ylmethyl]phenyl}methanone (5e)

Starting from **4b** (2.1 g, 5.1 mmol), the title compound was prepared following the above described procedure and was obtained as a white solid (1.86 g, 88%); ¹H NMR (300 MHz, CDCl₃) δ 7.29-7.20 (m, 6H), 5.69 (s, 1H), 3.98 (bs, 1H), 3.41 (s, 2H), 3.34 (m, 4H), 2.30 (m, 4H), 1.41 (s, 9H); ESI MS *m*/*z* 416 (M+H)⁺.

### [3-Chloro-4-(pyrrolidin-1-ylmethyl)phenyl](3-chlorophenyl)methanone (5f)

Starting from **4c** (5.8 g, 17 mmol) and pyrrolidine, the title compound was prepared following the procedure described for **5a** and after flash chromatography (CHCl₃/MeOH 20:1) was obtained as a white solid (5.1 g, 90%); ¹H NMR (CDCl₃) δ 7.78 (m, 2H), 7.65-7.47 (m, 4H), 7.44-7.42 (m, 1H), 3.82 (s, 2H), 2.63 (m, 4H), 1.84 (m, 4H); ESI MS *m*/*z* 334 (M+H)⁺.

### [3-Chloro-4-(morpholin-4-ylmethyl)phenyl](3-chlorophenyl)methanone (5g)

Starting from **4c** (5.5 g, 16 mmol) and morpholine, the title compound was prepared following the procedure described for **5a** and after flash chromatography (CHCl₃/MeOH 20:1) was obtained as a white solid (5.5 g, 98%); ¹H NMR (CDCl₃) δ 7.79-7.58 (m, 6H), 7.47-7.45 (m, 1H), 3.75 (m, 4H), 3.69 (s, 2H), 2.56 (m, 4H); ESI MS *m*/*z* 350 (M+H)⁺.

### (4-Chlorophenyl)[3-fluoro-4-(pyrrolidin-1-ylmethyl)phenyl]methanone (5h)

Starting from **4d** (2.0 g, 6.1 mmol) and pyrrolidine, the title compound was prepared following the procedure described for **5a** and after flash chromatography (CHCl₃/MeOH 9:1) was obtained as a white solid (1.7 g, 90%); ¹H NMR (300 MHz, CDCl₃) δ 7.71 (d, 2H, *J* = 8.2 Hz), 7.55-7.40 (m, 4H), 7.26 (s, 1H), 3.72 (s, 2H), 2.55 (m, 4H), 1.77 (m, 4H); ESI MS *m*/*z* 318(M+H)⁺.

### (4-Chlorophenyl)[4-(1H-imidazol-1-yl)methylphenyl]methanone (5i)

To a suspension of 310 mg (12.9 mmol) of NaH (as 60% oil dispersion) in 30 mL of dry DMF, imidazole (881 mg, 12.9 mmol) was added and the resulting mixture was stirred for 1 h at rt. Thereafter, the mixture was cooled to 0°C and **4b** (2.0 g, 6.5 mmol) was added, the solution was stirred at rt for 1h and the solvent was removed. H₂O (20 mL) was added to the solid residue, the aqueous phase was extracted with chloroform (40 mL X 3), the combined organic layers were dried over Na₂SO₄, filtered, and the solvent evaporated. The crude residue was purified by flash chromatography (EtOAc/*n*-Hexane 1:10) to afford the title compound as a white solid (1.4 g, 75%). ¹H-NMR (CDCl₃) δ 7.75-7.68 (m, 4H), 7.57 (m, 1H), 7.43 (d, 2H, *J*= 8.5 Hz), 7.23 (d, 2H, *J=* 8.2 Hz), 7.11 (m, 1H), 6.92 (m, 1H), 5.2 (s, 2H). ESI-MS *m*/*z* 297 (M+H)⁺.

### [3-Chloro-4-(pyrrolidin-1-ylmethyl)phenyl](3-chlorophenyl)methanone oxime (7f)

To a solution of **5f** (3.3 g, 10.1 mmol) in methanol (150 mL), hydroxylamine hydrochloride (1.6 g, 23.3 mmol) and BaCO₃ (4.6 g, 23.3 mmol) were added and the mixture was heated under reflux for 18 h. Thereafter, the white precipitate was removed by filtration and the solvent was evaporated under reduced pressure. After evaporation of the solvent, the crude oxime was obtained as colourless oil and used without further purification; ESI MS *m*/*z* 349 (M+H)⁺.

### [3-chloro-4-(morpholin-4-ylmethyl)phenyl](3-chlorophenyl)methanone oxime (7g)

The title compound was prepared following the above described procedure starting from **5g** (2.9 g, 8.3 mmol). The crude product was used in the next step without further purification; ESI MS *m*/*z* 365 (M+H)⁺.

### (4-Chlorophenyl)[3-fluoro-4-(pyrrolidin-1-ylmethyl)phenyl]methanone oxime (7h)

The title compound was prepared following the above described procedure starting from **5h** (2.5 g, 12.6 mmol). The crude product was used in the next step without further purification; ESI MS *m*/*z* 347 (M+H)⁺.

### (±)-1-(3-Chlorophenyl)-1-[4-(pyrrolidin-1-ylmethyl)phenyl]methylamine (6a)

To a mixture of **5a** (2.8 g, 9.4 mmol) in a 2.0 M solution of ammonia in EtOH (24 mL, 50 mmol), titanium(IV) isopropoxide (5.5 mL, 18.8 mmol) was added and the reaction mixture was allowed to stir for 16 h. Subsequently, sodium borohydride (0.53 g, 14.0 mmol) was added and the mixture was stirred at rt for 3 h and then poured into a 2.0 M solution of ammonium hydroxide. The inorganic precipitate was filtered off and the aqueous phase was extracted with CHCl₃. The organic extracts were washed with brine, dried over Na₂SO₄ and the solvent was removed. The residue was purified by flash-chromatography eluting 5% MeOH in CHCl₃ to afford 0.83 g (30%) of **7a** as colourless oil; ¹H NMR (200 MHz, CDCl₃) δ 7.40-7.36 (m, 1H), 7.28-7.21 (m, 4H), 7.21-7.17 (m, 3H), 5.14 (s, 1H), 3.61 (s, 2H), 2.55-2.53 (m, 4H), 2.28 (bs, 2H), 1.88-1.85 (m, 4H); ESI MS *m*/*z* 301 (M+H)⁺.

### (±)-1-(4-Chlorophenyl)-1-[4-(pyrrolidin-1-ylmethyl)phenyl]methylamine (6b)

Starting from **5b,** the title compound was prepared following the procedure described for compound **6a** and was obtained as a yellow viscous oil; ¹H NMR (300 MHz, CDCl₃) δ 7.34-7.21 (m, 8H), 5.17 (s, 1H), 3.58 (s, 2H), 2.52-2.46 (m, 4H), 2.0 (bs, 2H), 1.90-1.72 (m, 4H); ESI MS *m*/*z* 301 (M+H)⁺.

### (±)-1-(3-Chlorophenyl)-1-[4-(morpholin-4-ylmethyl)phenyl]methylamine (6c)

Starting from **5c,** the title compound was prepared following the procedure described for compound **6a** and was obtained as a yellow viscous oil; ¹H NMR (300 MHz, CDCl₃) δ 7.35-7.11 (m, 8H), 5.07 (s, 1H), 3.62-3.59 (m, 4H), 3.39 (s, 2H), 2.36-2.33 (m, 4H), 2.04 (bs, 2H). ESI MS *m*/*z* 317 (M+H)⁺.

### (±)-1-(3-Chlorophenyl)-1-[3-chloro-4-(pyrrolidin-1-ylmethyl)phenyl]methylamine (6f)

To suspension of LiAlH₄ (1.0 g, 28.0 mmol) in dry THF (30 mL), heated under reflux, a solution of **7f** (3.2 g, 9.35 mmol) in THF (15 mL) was added dropwise. The resulting mixture was heated under reflux for 1 h, then cooled to 0°C and quenched with ethanol and H₂O. Thereafter, the solvent was evaporated and the residue was suspended in 20 mL of H₂O and the aqueous layer was extracted with CHCl₃ (3 × 40 ml). The combined organic layers were dried over Na₂SO₄ and evaporated under reduced pressure. The crude residue was purified by flash chromatography (CHCl₃/MeOH 20:1) to give the title compound as a yellow oil (1.5 g, 50%); ¹H NMR (CDCl₃) δ 7.44-7.24 (m, 3H), 7.23-7.19 (m, 4H), 5.13 (s, 1H), 3.72 (s, 2H), 2.57 (m, 4H), 1.79 (m, 6H); ESI MS *m*/*z* 335 (M+H)⁺.

### (±)-1-(3-Chlorophenyl)-1-[3-chloro-4-(morpholin-4-ylmethyl)phenyl]methylamine (6g)

The title compound was prepared from **7g** (2.9 g, 8.0 mmol) following the procedure described for **6f** and was obtained as a yellow oil (1.7 g, 62%). ¹H NMR (CDCl₃) δ 7.43-7.38 (m, 3H), 7.26-7.20 (m, 4H), 5.12 (s, 1H), 3.71-3.68 (m, 4H), 3.57 (s, 2H), 2.51-2.48 (m, 4H), 1.92 (bs, 2H); ESI MS *m*/*z* 351 (M+H)⁺.

### (±)-1-(4-Chlorophenyl)-1-[3-fluoro-4-(pyrrolidin-1-ylmethyl)phenyl]methylamine (6h)

The title compound was prepared from **7h** (2.0 g, 5.99 mmol) following the procedure described for **6f** and was obtained as a yellow oil (1.62 g, 85%). ¹H NMR (300 MHz, CDCl₃) δ 7.44-7.24 (m, 3H), 7.23-7.08 (m, 4H), 5.11 (s, 1H), 3.69 (s, 2H), 2.69 (m, 4H), 2.94 (m, 6H); ESI MS *m*/*z* 320 (M+H)⁺.

### (±)-(4-Chlorophenyl)[4-(morpholinomethyl)phenyl]methanol (8d)

To a stirred solution of **5d** (0.58 g, 1.84 mmol) in ethanol (10 mL), sodium borohydride (90.0 mg, 2.5 mmol) was added at 0 °C and the resulting mixture was allowed to stir for 1 h at 0 °C. Thereafter, the reaction mixture was quenched by dropwise addition of water (5 mL) and the solvent was evaporated under reduced pressure. The residue was treated with water and extracted with chloroform (3 × 20 mL). The combined organic layers were washed with brine, dried over Na₂SO₄ and evaporated. The residue was chromatographed (6% methanol in chloroform) to afford **8d** as a brown oil (0.51 g, 87%);¹H NMR (300 MHz, CDCl₃) δ 7.25-7.23 (m, 8H), 5.67 (s, 1H), 4.03 (bs, 1H), 3.57 (m, 4H), 3.41 (s, 2H), 3.38 (m, 4H); ESI MS *m*/*z* 318 (M+H)⁺.

### (±)-(4-Chlorophenyl)[4-(1-tert-butoxycarbonylpiperazin-4-ylmethyl)phenyl]methanol (8e)

Starting from **5e** (2.1 g, 5.1 mmol), the title compound was prepared following the procedure described for **8d** and was obtained as a white solid (1.86 g, 88%); ¹H NMR (300 MHz, CDCl₃) δ 7.29-7.20 (m, 8H), 5.69 (s, 1H), 3.98 (bs, 1H), 3.41 (s, 2H), 3.34 (m, 4H), 2.30 (m, 4H), 1.41 (s, 9H); ESI MS *m*/*z* 417 (M+H)⁺.

### (±)-(4-chlorophenyl)[4-(1H-imidazol-1-yl)methylphenyl]methanol (8i)

Starting from **5i** (831 mg, 2.8 mmol), the title compound was prepared following the procedure described for **8d** and was obtained as a white solid (795 mg, 95 %); ¹H-NMR (CDCl₃) δ 7.34-7.26 (m, 7H), 7.05-7.02 (m, 2H), 6.88 (m, 1H), 6.81 (m, 1H), 5.79 (s, 1H), 5.04 (s, 2H); ESI-MS *m*/*z* 299 (M+H)⁺.

### (±)-7-Chloro-N-{(3-chlorophenyl)[4-(pyrrolidin-1-ylmethyl)phenyl]methyl}-4-aminoquinoline (9a) -

To a solution of **7a** (0.83g, 2.8 mmol) in 2-ethoxyethanol (10 mL), 4,7-dichloroquinoline (0.57 g, 2.9 mmol) and pyridium chloride (0.35 g, 3.0 mmol) were added. The resulting mixture was heated to 135°C for 5 h and then was cooled to rt. Triethylamine (0.5 mL) was added and the solvent was evaporated under reduced pressure. The residue was purified by flash-chromathography (5% MeOH in CHCl₃) to afford **7a** as a white low-melting solid (0.9g, 70%). ¹H NMR (300 MHz, CDCl₃) δ 8.44 (d, 1H, *J* = 5.3 Hz), 7.98 (d, 1H, *J* = 2.0 Hz), 7.72 (d, 1H, *J* = 8.8 Hz), 7.41-7.26 (m, 9H), 6.23 (d, 1H, *J* = 5.3 Hz), 5.68 (d, 1H, *J* = 4.4 Hz), 5.39 (d, 1H, *J* = 4.4 Hz), 3.68 (s, 2H), 2.61-2.59 (m, 4H), 1.85-1.81 (m, 4H).

### (±)-7-Chloro-N-{(3-chlorophenyl)[4-(morpholin-4-ylmethyl)phenyl]methyl}-4-aminoquinoline (9b)

Starting from **7c** and 4,7-dichloroquinoline, the title compound was prepared following the procedure described for **9a;** ¹H NMR (300 MHz, CDCl₃) ^{δ} 8.45 (d, 1H, *J* = 5.3 Hz), 8.0 (s, 1H), 7.72 (d, 1H, *J* = 9.1 Hz), 7.41-7.26 (m, 9H), 6.25 (d, 1H, *J* = 5.6 Hz), 5.70 (d, 1H, *J* = 4.1 Hz), 5.41 (d, 1H, *J* = 3.2 Hz), 3.72-3.70 (m, 4H), 3.50 (s, 2H), 2.47-2.45 (m, 4H).

### (±)-7-Chloro-N-{[3-chloro-4-(pyrrolidin-1-ylmethyl)phenyl](3-chlorophenyl)methyl}-4-aminoquinoline (9c)

Starting from **7f** (156 mg, 0.467 mmol) and 4,7-dichloroquinoline, the title compound was prepared following the procedure described for **9a** and was obtained as a white solid (120 mg, 52%); ¹H NMR (300 MHz, CDCl₃) δ 8.45 (d, 1H, *J* = 5.3 Hz), 7.99 (s, 1H), 7.73 (d, 1H, *J* = 8.8 Hz), 7.52 (d, 1H, *J*= 7.9 Hz), 7.40 (d, 1H, *J* = 8.2 Hz), 7.30-7.20 (m, 6H), 6.22 (d, 1H, *J* = 5.3 Hz), 5.65 (d, 1H, *J* = 4.1 Hz), 5.37 (d, 1H, *J* = 4.1 Hz), 3.74 (s, 2H), 2.60 (m, 4H), 1.81 (m, 4H); ESI MS *m*/*z* 496 (M+H)⁺.

### (±)-7-Chloro-N-{[3-chloro-4-(morpholin-4-ylmethyl)phenyl](3-chlorophenyl)methyl}-4-aminoquinoline (9d)

Starting from **7g** (260 mg, 0.74 mmol) and 4,7-dichloroquinoline, the title compound was prepared following the procedure described for **9a** and was obtained as a white solid (220 mg, 58%); ¹H NMR (CDCl₃) δ 8.35 (d, 1H, *J* = 5.3 Hz), 7.88-7.83 (m, 2H), 7.46 (d, 1H, *J* = 7.9 Hz), 7.30-7.19 (m, 7H), 6.19 (d, 1H, *J* = 5.3 Hz), 5.84 (d, 1H, *J* = 4.1 Hz), 5.65 (d, 1H, *J* = 4.1 Hz), 3.66 (m, 4H), 3.55 (s, 2H), 2.47 (m, 4H); ESI MS *m*/*z* 512 (M+H)⁺.

### (±)-7-Chloro-N-{(4-chlorophenyl)[4-(pyrrolidin-1-ylmethyl)phenyl]methyl}-4-aminoquinoline (9e)

Starting from **7b** and 4,7-dichloroquinoline, the title compound was prepared following the procedure described for **9a;** ¹H NMR (300 MHz, CDCl₃) δ 8.42 (d, 1H, *J* = 5.3 Hz), 7.97 (d, 1H, *J* = 2.0 Hz), 7.72 (d, 1H, *J* = 8.8 Hz), 7.40-7.26 (m, 9H), 6.23 (d, 1H, *J* = 5.3 Hz), 5.71 (d, 1H, *J* = 4.1 Hz), 5.41 (d, 1H, *J* = 4.1 Hz), 3.63 (s, 2H), 2.55-2.54 (m, 4H), 1.82-1.78 (m, 4H).

### (±)-7-Chloro-N-{(4-chlorophenyl)[4-(morpholin-4-ylmethyl)phenyl]methyl}-4-aminoquinoline (9f)

To a solution of **8d** (0.25 g, 0.78 mmol) in dry DCM (8 mL) was added a solution of SOCl₂ (451 µL, 6.18 mmol) in dry DCM (4 mL) at 0 °C, and the mixture was stirred at 0 °C for 20 min and thereafter heated to 45 °C for 4 h. The volatiles were removed and the residue was treated with dry MeCN (3 × 4 mL) and concentrated under reduced pressure in order to remove residual SOCl₂. The resulting hydrochloride salt was suspended in dry MeCN (12 mL) and and to this solution was added a solution containing triethylamine (430 µL, 3.0 mmol) and 4-amino-7-chloroquinoline (0.16 g, 0.94 mmol) at 0 °C. Thereafter, the reaction mixture was heated to 80 °C for 6h. The solvent was evaporated under reduced pressure and the residue was treated with water and extracted with ethyl acetate (4 × 30 mL). The combined organic layers were dried over Na₂SO₄ and concentrated *in vacuo.* The crude residue was purified by flash column chromatography (2% MeOH in CHCl₃) to afford **9f** as a light yellow solid (0.11 g, 32%); ¹H NMR (300 MHz, CDCl₃) δ 8.43 (d, 1H, *J* = 5.2 Hz), 7.98 (s, 1H), 7.71 (d, 1H, *J* = 9.0 Hz), 7.39-7.26 (m, 9H), 6.23 (d, 1H, *J* = 5.2 Hz), 5.70 (d, 1H, *J* = 4.1 Hz), 5.38 (d, 1H, *J* = 3.8 Hz), 3.70 (m, 4H), 3.49 (s, 2H), 2.44 (m, 4H); ESI MS m/z 478 (M+H)⁺.

### (±)-7-Chloro-N-{(4-chlorophenyl)[4-(piperazin-1-ylmethyl)phenyl]methyl}-4-aminoquinoline (9g)

Starting from **8e** (0.12 g, 0.28 mmol) and 4,7-dichloroquinoline, the title compound was prepared following the procedure described for **9f** and was obtained as a white solid (90.0 mg, 68%); ¹H NMR (300 MHz, CDCl₃) δ 8.40 (d, 1H, *J* = 5.2 Hz), 7.95 (d, 1H, *J* = 2.0 Hz), 7.72 (d, 1H, *J* = 8.7 Hz), 7.36-7.23 (m, 9H), 6.21 (d, 1H, *J* = 5.2 Hz), 5.68 (d, 1H, *J* = 4.3 Hz), 5.46 (d, 1H, *J* = 4.3 Hz), 3.47 (s, 2H), 2.86 (m, 4H), 2.40 (m, 4H), 1.84 (s, 1H); ESI MS *m*/*z* 477 (M+H)⁺.

### (±)-7-Chloro-N-{(4-chlorophenyl)[3-fluoro-4-(pyrrolidin-1-ylmethyl)phenyl]methyl}-4-aminoquinoline (9h)

Starting from **7h** (100 mg, 0.32 mmol) and 4,7-dichloroquinoline, the title compound was prepared following the procedure described for **9a** and was obtained as a white solid (120 mg, 78%); ¹H NMR (300 MHz, CDCl₃) δ 8.41 (d, 1H, *J* = 4.7 Hz), 7.95 (s, 1H), 7.75 (d, 1H, *J* = 8.8 Hz), 7.43-7.28 (m, 6H) 7.09 (d, 1H, *J* = 7.6 Hz), 6.98 (d, 1H, *J* = 10.3 Hz), 6.21 (d, 1H, *J* = 4.7 Hz), 5.67 (s, 1H), 5.51 (s, 1H), 3.65 (s, 2H), 2.54 (m, 4H), 1.77 (m, 4H); ESI MS *m*/*z* 480 (M+H)⁺.

### (±)-N-{[3-Chloro-4-(pyrrolidin-1-ylmethyl)phenyl](3-chlorophenyl)methyl}-7-trifluoromethyl-4-aminoquinoline (9i)

Starting from **7f** (573 mg, 1.71 mmol) and 7-trifluoromethyl-4-chloroquinoline, the title compound was prepared following the procedure described for **9a** and was obtained as a white solid (420 mg, 46%). ¹H NMR (CDCl₃) δ 8.48 (d, 1H, *J* = 5.3 Hz), 8.25 (s, 1H), 8.01 (d, 1H, *J* = 8.8 Hz), 7.57 (d, 1H, *J* = 8.8 Hz), 7.49 (d, 1H, *J* = 7.9 Hz) 7.31-7.20 (m, 6H), 6.31 (d, 1H, *J* = 5.3 Hz), 5.75 (d, 1H, *J* = 4.1 Hz), 5.68 (d, 1H, *J*= 4.1 Hz), 3.71 (s, 2H), 2.57 (m, 4H), 1.78 (m, 4H); ESI MS *m*/*z* 530 (M+H)⁺.

### (±)-N-{[3-Chloro-4-(morpholin-4-ylmethyl)phenyl](3-chlorophenyl)methyl}-7-trifluoromethyl-4-aminoquinoline (9j)

Starting from **7g** (293 mg, 0.83 mmol) and 7-trifluoromethyl-4-chloroquinoline, the title compound was prepared following the procedure described for **9a** and was obtained as a white solid (250 mg, 55%). ¹H NMR (CDCl₃) δ 8.56 (d, 1H, *J* = 5.3 Hz), 8.31 (s, 1H), 7.91 (d, 1H, *J* = 8.8 Hz), 7.63 (d, 1H, *J* = 9.1 Hz), 7.52 (d, 1H, *J* = 8.2 Hz), 7.35-7.22 (m, 6H), 6.32 (d, 1H, *J* = 5.3 Hz), 5.67 (d, 1H, *J* = 4.1 Hz), 5.40 (d, 1H, *J* = 4.1 Hz), 3.74-3.71 (m, 4H), 3.61 (s, 2H), 2.54-2.51 (m, 4H); ESI MS *m*/*z* 546 (M+H)⁺.

### (±)-N-{(4-Chlorophenyl)[4-(pyrrolidin-1-ylmethyl)phenyl]methyl}-7-trifluoromethyl-4-aminoquinoline (9k)

Starting from **7b** and 7-trifluoro-4-chloroquinoline, the title compound was prepared following procedure described for **9a;** ¹H NMR (300 MHz, CDCl₃) δ 8.52 (d, 1H, *J* = 5.3 Hz), 8.29 (s, 1H), 7.91 (d, 1H, *J* = 8.8 Hz), 7.61 (dd, 1H, *J* = 1.8, 8.8 Hz), 7.39-7.28 (m, 9H), 6.33 (d, 1H, *J* = 4.4 Hz), 5.48 (d, 1H, *J* = 4.4 Hz), 3.65 (s, 2H), 2.57-2.55 (m, 4H), 1.84-1.80 (m, 4H).

### (±)-N-{(4-Chlorophenyl)[3-fluoro-4-(pyrrolidin-1-ylmethyl)phenyl]methyl}-7-trifluoromethyl-4-aminoquinoline (91)

Starting from **7h** (196 mg, 0.62 mmol) and 7-trifluoromethyl-4-chloroquinoline, the title compound was prepared following the procedure described for **9a** and was obtained as a white solid (98 mg, 35%); ¹H NMR (300 MHz, CDCl₃) δ 8.53 (d, 1H, *J* = 5.0 Hz), 8.30 (s, 1H), 7.91 (d, 1H, *J* = 7.4 Hz), 7.62 (d, 1H, *J* = 8.8Hz) 7.43-7.26 (m, 5H), 7.11 (d, 1H, *J* = 7.9 Hz), 7.0 (d, 1H, *J* = 10.3 Hz), 6.32 (d, 1H, *J* = 5.0 Hz), 5.71 (d, 1H, *J* = 4.1), 5.46 (d, 1H, *J* = 3.8 Hz), 3.67 (s, 2H), 2.56 (m, 4H), 1.79 (m, 4H); ESI MS *m*/*z* 514 (M+H)⁺.

### (±)-6-Methoxy-N-{(3-chlorophenyl)[4-(pyrrolidin-1-ylmethyl)phenyl]methyl}-4-aminoquinoline (9m)

Starting from **7a** (178 mg, 0.56 mmol) and 6-methoxy-4-chloroquinoline, the title compound was prepared following the procedure described for **9a** and was obtained as a white solid (140 mg, 48%); ¹H NMR (300 MHz, CDCl₃) δ 8.28 (d, 1H, *J* = 5.3 Hz), 7.91 (d, 1H, *J* = 9.4 Hz), 7.36-7.25 (m, 9H), 7.08 (s, 1H), 6.22 (d, 1H, *J* = 5.3 Hz), 5.70 (d, 1H, *J* = 4.1 Hz), 5.47 (d, 1H, *J* = 3.8 Hz), 3.85 (s, 3H), 3.62 (s, 2H), 2.55 (m, 4H), 1.79 (m, 4H); ¹³C NMR (300 MHz, CDCl₃) δ 157.3, 148.4, 148.0, 143.9, 143.4, 139.7, 139.4, 135.0, 131.4, 130.5, 130.0, 128.2, 127.9, 127.7, 125.9, 120.8, 119.6, 101.5, 99.7, 62.0, 60.4, 56.0, 54.5, 23.7; ESI MS *m*/*z* 458 (M+H)⁺.

### (±)-N-{(3-Chlorophenyl)[4-(morpholin-4-ylmethyl)phenyl]methyl}-6-methoxy-4-aminoquinoline (9n)

Starting from **7c** (910 mg, 2.87 mmol) and 6-methoxy-4-chloroquinoline, the title compound was prepared following the procedure described for **9a** and was obtained as a white solid (550 mg, 40%); ¹H NMR (CDCl₃) δ 8.34 (d, 1H, *J* = 5.0 Hz), 7.94 (d, 1H, *J* = 9.4 Hz), 7.36-7.26 (m, 9H), 7.02 (s, 1H), 6.23 (d, 1H, *J* = 5.0 Hz), 5.69 (d, 1H, *J* = 3.8 Hz), 5.24 (d, 1H, *J* = 3.8 Hz), 3.84 (s, 3H), 3.69 (m, 4H), 3.47 (s, 2H), 2.43 (m, 4H); ¹³C NMR (CDCl₃) δ 157.3, 148.7, 147.6, 144.0, 143.5, 139.9, 138.4, 135.2, 131.8, 130.6, 130.2, 128.3, 127.7, 127.7, 125.7, 120.3, 119.7, 101.6, 99.7, 67.2, 63.2, 62.0, 56.0, 53.9; ESI MS *m*/*z* 474 (M+H)⁺.

### (±)-N-{[3-Chloro-4-(pyrrolidin-1-ylmethyl)phenyl](3-chlorophenyl)methyl}-6-methoxy-4-aminoquinoline (9o)

Starting from **7f** (910 mg, 2.87 mmol) and 6-methoxy-4-chloroquinoline, the title compound was prepared following the procedure described for **9a** and was obtained as a white solid (250 mg, 30%); ¹H NMR (CDCl₃) δ 8.37 (d, 1H, *J* = 5.3 Hz), 7.95 (d, 1H, *J* = 9.4 Hz), 7.51 (d, 1H, *J* = 7.9 Hz), 7.36-7.22 (m, 7H), 6.98 (d, 1H, *J* = 2.0 Hz), 6.23 (d, 1H, *J* = 5.3 Hz), 5.66 (d, 1H, *J* = 3.8 Hz), 5.09 (d, 1H, *J* = 3.8 Hz),3.93 (s, 3H), 3.74 (s, 2H), 2.61 (m, 4H), 1.81 (m, 4H); ESI MS *m*/*z* 492 (M+H)⁺.

### (±)-6-Methoxy-N-{(4-chlorophenyl)[4-(pyrrolidin-1-ylmethyl)phenyl]methyl}-4-aminoquinoline (9p)

Starting from **7b** and 6-methoxy-4-chloroquinoline, the title compound was prepared following the procedure described for **9a;** ¹H NMR (300 MHz, CDCl₃) δ 8.31 (d, 1H, *J* = 5.0 Hz), 7.93 (d, 1H, *J* = 9.1 Hz), 7.38-7.26 (m, 9H), 7.03 (d, 1H, *J* = 2.6 Hz, 1H), 6.22 (d, 1H, *J* = 5.3 Hz), 5.71 (d, 1H, *J* = 4.1 Hz), 5.32 (d, 1H, *J* = 4.1 Hz), 3.88 (s, 3H), 3.65 (s, 2H), 2.57-2.55 (m, 4H), 1.84-1.79 (m, 4H).

### (±)-N-{(4-Chlorophenyl)[4-(morpholin-4-ylmethyl)phenyl]methyl}-6-methoxy-4-aminoquinoline (9q)

Starting from **8d** (0.25 g, 0.78 mmol) and 6-methoxy-4-aminoquinoline, the title compound was prepared following the procedure described for **9f** and was obtained as brown oil (52 mg, 14%); ¹H NMR (300 MHz, CDCl₃) δ 8.32 (d, 1H, *J* = 5.2 Hz), 7.91 (d, 1H, *J* = 9.3 Hz), 7.34-7.26 (m, 9H), 7.03 (s, 1H), 6.23 (d, 1H, *J* = 5.2 Hz), 5.71 (d, 1H, *J* = 3.8 Hz), 5.28 (d, 1H, *J* = 3.5 Hz), 3.85 (s, 3H), 3.70 (m, 4H), 3.49 (s, 2H), 2.44 (m, 4H); ESI MS *m*/*z* 474 (M+H)⁺.

### (±)-N-{(4-Chlorophenyl)[4-(piperazin-1-ylmethyl)phenyl]methyl}-6-methoxy-4-aminoquinoline (9r)

Starting from **8e** (80 mg, 0.14 mmol) and 6-methoxy-4-aminoquinoline, the title compound was prepared following the procedure described for **9f** and was obtained as a light yellow oil (50 mg, 75.75%); ¹H NMR (300 MHz, CDCl₃) δ 8.31 (d, 1H, *J* = 4.9 Hz), 7.90 (d, 1H, *J* = 9.0 Hz), 7.32-7.25 (m, 9H), 7.03 (s, 1H), 6.21 (d, 1H, *J* = 4.9 Hz), 5.69 (d, 1H, *J* = 4.1 Hz), 5.27 (s, 1H), 3.87 (s, 3H), 3.47 (s, 2H), 2.87 (m, 4H), 2.40 (m, 4H), 1.90 (s, 1H); ESI MS *m*/*z* 473 (M+H)⁺.

### (±)-6-Chloro-N-{(4-chlorophenyl)[4-(pyrrolidin-1-ylmethyl)phenyl]methyl}-2-methoxy-9-aminoacridine (9s)

Starting from **7b** and 6,9-dichloro-2-methoxyacridine, the title compound was prepared following the procedure described for **9a;** ¹H NMR (300 MHz, CDCl₃) δ 8.07 (d, 1H, *J* = 2.0 Hz), 7.97 (d, 1H, *J* = 9.7 Hz), 7.48 (d, 1H, *J* = 9.4 Hz), 7.35-7.29 (m, 7H), 7.20 (d, 2H, *J* = 7.9 Hz), 7.14 (dd, 1H, *J* = 2.0, 9.4 Hz), 6.84 (d, 1H, *J* = 2.6 Hz), 5.87 (d, 1H, *J* = 8.5 Hz), 4.80 (d, 1H, *J* = 8.8 Hz), 3.60 (s, 2H), 3.42 (s, 3H), 2.51 - 2.46 (m, 4H), 1.81-1.77 (m, 4H).

### (±)-7-Chloro-N-{[4-(1H-imidazol-1-yl)methylphenyl](4-chlorophenyl)methyl}-4-aminoquinoline (9t)

Starting from **8i** (795 mg, 2.67 mmol) and 7-chloro-4-aminoquinoline, the title compound was prepared following the procedure described for **9f** and was obtained as a white solid (550 mg, 45 %); ¹H-NMR (CDCl₃) δ 8.34 (d, 1H, *J* = 5.3 Hz), 7.89 (d, 1H, *J* = 2.1 Hz), 7.85 (d, 1H, *J* = 9.1 Hz), 7.39 (s, 1H), 7.34-7.19 (m, 8H), 7.09 (d, 1H, *J* = 7.9 Hz), 6.98 (m, 1H), 6.85 (m, 1H), 6.17 (d, 1H, *J* = 5.3 Hz), 5.94 (d, 1H, *J* = 4.7 Hz), 5.70 (d, 1H, *J* = 4.7 Hz), 5.05 (s, 2H); ESI-MS *m*/*z* 459 (M+H)⁺.

### Example 2

### Synthesis of Derivatives 15a-d according to Scheme 2

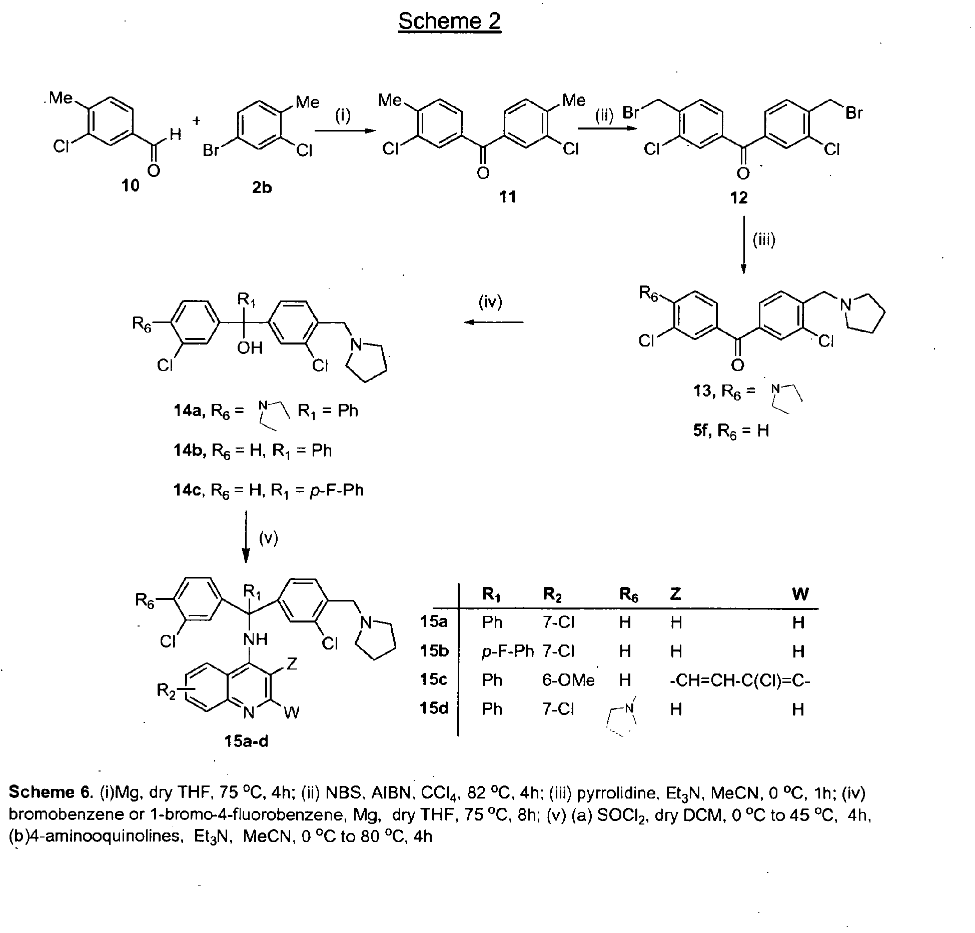

### bis(3-Chloro-4-methylphenyl)methanone (11)

Starting from **2b** (2.0 g, 9.73 mmol) and **10** (3.0 g, 19.46 mmol), the title compound was prepared using magnesium turnings (0.24 g, 9.73 mmol) as described for **3a,** and was obtained as a white solid (2.5 g, 92%); ¹H NMR (300 MHz, CDCl₃) δ 7.75 (d, 2H, J = 1.4 Hz), 7.56-7.52 (m, 2H), 7.34-7.31 (m, 2H), 2.44 (s, 6H).

### bis(4-Bromomethyl-3-chlorophenyl)methanone (12)

To a solution of **11** (2.7 g, 9.67 mmol) in CCl₄ (30 mL), NBS (3.78 g, 21.27 mmol) and AIBN (catalytic amount) were added. The solution was heated under reflux for 4 h. After cooling to rt, the succinimide was filtered off and the solvent was evaporated. The residue was purified by flash chromatography (2% ethyl acetate in hexane) to afford **12** as a white solid (1.8 g, 42.65%); ¹H NMR (300 MHz, CDCl₃) δ 7.79 (m, 2H), 7.64-7.55 (m, 4H), 4.61 (s, 4H).

### bis[3-Chloro-4-(pyrrolidin-1-ylmethyl)phenyl]methanone (13)

To a stirred solution of **12** (1.8 g, 4.12 mmol) in dry MeCN (20 mL), cooled to 0°C, pyrrolidine (0.83 mL, 9.88 mmol) and triethylamine (2.3 mL, 16.45 mmol) were added and the resulting mixture was allowed to stir for 1 h at 0 °C. Thereafter, the reaction mixture was quenched with 2 mL of water and the solvent was evaporated under reduced pressure. The residue was treated with water and extracted with DCM (3 × 30 mL). The combined organic layers were washed with brine, dried over Na₂SO₄ and evaporated. The residue was chromatographed (2% methanol in chloroform) to afford **13** as a yellow viscous oil (1.5 g, 88%); ¹H NMR (400 MHz, CDCl₃) δ 7.77 (m, 2H), 7.60-7.56 (m, 4H), 3.97 (s, 4H), 2.57 (m, 8H), 1.76 (m, 8H); ESI MS *m*/*z* 417 (M+H)⁺.

### bis[3-Chloro-4-(pyrrolidin-1-ylmethyl)phenyl]phenylmethanol (14a)

Starting from **13** (0.60 g, 1.43 mmol) and bromobenzene (0.22 mL, 2.15 mmol), the title compound was prepared using magnesium turnings (52.2 mg, 2.15 mmol) as described for **3a,** and was obtained as a light yellow oil (0.57 g, 79%); ¹H NMR (300 MHz, CDCl₃) δ 7.37-7.20 (m, 9H), 7.09-7.06 (m, 2H), 3.96 (bs, 1H), 3.70 (s, 4H), 2.54 (m, 8H), 1.77 (m, 8H); ESI MS *m*/*z* 495 (M+H)⁺.

### (±)-(4-Chlorophenyl)[4-(pyrrolidin-1-ylmethyl)phenyl]phenylmethanol (14b)

Starting from **5f** (1.25 g, 4.2 mmol) and bromobenzene (4.2 mmol), the title compound was prepared using magnesium turnings (4.2 mmol) as described for **3a,** and was obtained as a brown oil (1.20 g, 76%); ¹H NMR (300 MHz, CDCl₃) δ 7.29-7.16 (m, 13H), 3.89 (bs, 1H), 3.58 (s, 2H), 2.49 (m, 4H), 1.73 (m, 4H); ESI MS *m*/*z* 378 (M+H)⁺.

### (±)-(4-Chlorophenyl)(4-fluorophenyl)[4-(pyrrolidin-1-ylmethyl)phenyl]methanol (14c)

Starting from **5f** (1.10 g, 3.7 mmol) and 1-bromo-4-fluorobenzene (3.7 mmol), the title compound was prepared using magnesium turnings (3.7 mmol) as described for **3a** and was obtained as a brown oil (0.94 g, 65%); ¹H NMR (300 MHz, CDCl₃) δ 7.33-7.16 (m, 10H), 7.01-6.99 (m, 2H), 3.66 (s, 2H), 2.78 (bs, 1H), 2.58 (m, 4H), 1.83-1.82 (m, 4H); ESI MS *m*/*z* 396 (M+H)⁺.

### (±)-7-Chloro-N-{(4-chlorophenyl)[4-(pyrrolidin-1-ylmethyl)phenyl]phenyl methyl}-4-aminoquinoline (15a)

To a solution of **14b** (320 mg, 0.85 mmol) and a drop of N,N-DMF in dry DCM (15 mL), a solution of SOCl₂ (180 µL, 2.54 mmol) in dry DCM (10 mL) was added at 0°C, and the mixture was stirred at 0°C for 20 min, thereafter was heated to 45°C for 4 h. The volatiles were removed and the residue was treated with dry DCM (3 × 10 mL) and concentrated under reduced pressure in order to remove residual SOCl₂. The resulting hydrochloride salt was dissolved in THF (15 mL) and added to a refluxing mixture of sodium hydride (60% in mineral oil, 68.0 mg, 1.7 mmol) and 4-amino-7-chloroquinoline (0.31 mg, 1.7 mmol) in dry THF (20 mL). The resulting mixture was heated under reflux overnight and then was allowed to cool to rt. Subsequently, the reaction mixture was quenched with ice and the aqueous phase was extracted with chloroform. The organic extracts were washed with brine, dried and concentrated *in vacuo*. The residue was purified by flash-chromathography eluting with a 10% mixture of MeOH in chloroform to afford **15a** as a pale yellow oil (105 mg, 23%);¹H NMR (300 MHz, CDCl₃) δ 8.14 (d, 1H, *J* = 5.2 Hz), 7.95 (d, 1H, 2.3 Hz), 7.74 (d, 1H, *J* = 8.8 Hz), 7.40-7.26 (m, 14H), 6.24 (bs, 1H), 5.86 (d, 1H, *J* = 5.7 Hz), 3.71 (s, 2H), 2.67-2.65 (m, 4H), 1.88-1.83 (m, 4H).

### (±)-7-Chloro-N-{(4-chlorophenyl)(4-fluorophenyl)[4-(pyrrolidin-1-ylmethyl)phenyl]methyl}-4-aminoquinoline (15b)

Starting from 14c and 4-amino-7-chloroquinoline, the title compound was prepared following the procedure described for **15a** and was obtained as a brown oil; ¹H NMR (300 MHz, CDCl₃) δ 8.16 (d, 1H, *J* = 5.6 Hz), 7.96 (d, 1H, *J* = 2.1 Hz), 7.72 (d, 1H, *J* = 9.1 Hz), 7.41 (dd, 1H, *J* = 2.1, 8.8 Hz), 7.32-7.20 (m, 10H), 7.03-6.97 (m, 2H), 6.19 (bs, 1H), 5.84 (d, 1H, *J* = 5.6 Hz), 3.60 (s, 2H), 2.53-2.50 (m, 4H), 1.82-1.80 (m, 4H).

### (±)-N-((4-Chlorophenyl)[4-(pyrrolidin-1-ylmethyl)phenyl]phenylmethyl)-6-chloro-2-methoxy-9-aminoacridine (15c)

Starting from **14a** (0.13 g, 0.31 mmol), and 6-chloro-2-methoxyacridin-9-amine (90 mg, 0.35 mmol) in dry DMF, the title compound was prepared following the procedure described for **15a** and was obtained as a yellow oil (80 mg, 38%); ¹H NMR (300 MHz, CDCl₃) δ 7.91 (d, 1H, *J* = 2.0 Hz), 7.83 (d, 1H, *J* = 9.3 Hz), 7.50 (d, 1H, *J* = 9.3 Hz), 5.35-7.32 (m, 4H), 7.23-7.17 (m, 5H), 7.12-7.05 (m, 4H), 7.98-6.92 (m, 2H), 6.75 (d, 1H, *J* = 2.3 Hz), 5.40 (s, 1H), 3.46 (s, 2H), 3.38 (s, 3H), 2.32 (m, 4H), 1.74 (m, 4H); ESI MS *m*/*z* 618 (M+H)⁺.

### N-{bis[3-Chloro-4-(pyrrolidin-1-ylmethyl)phenyl]phenylmethyl}-7-chloro-4-aminoquinoline (15d)

Starting from **14a** (0.16 g, 0.33 mmol), and 4-amino-7-chloroquinoline (70 mg, 0.39 mmol), the title compound was prepared following the above described procedure and was obtained as a yellow oil (23 mg, 11 %); ¹H NMR (300 MHz, CDCl₃) δ 8.17 (d, 1H, *J* = 5.2 Hz), 7.97 (d, 1H, *J* = 2.0 Hz), 7.74 (d, 1H, *J* = 9.0 Hz), 7.44-7.40 (m, 3H), 7.32-7.31 (m, 7H), 7.19-7.15 (m, 2H), 6.17 (s, 1H), 5.88 (d, 1H, *J* = 5.5 Hz), 3.72 (s, 4H), 2.57 (m, 8H), 1.79 (m, 8H); ESI MS *m*/*z* 657 (M+H)⁺.

### Example 3

### In vitro activity of selected compounds

Some of the synthesized compounds were tested *in vitro* against two different strains of *P. falciparum,* namely 3D7, NF54 and D10 (CQ-S strain) and K1 and W2 (CQ-R strain). The pharmacological results are displayed in Table 2. In this table the values for CLT (clotrimazole) and CQ (chloroquine) are also reported.

**Table 2**

| **Cpd** | **IC₅₀ (nM)** | | | | | **TC₅₀ (**µ**M)** | | | |
|---|---|---|---|---|---|---|---|---|---|
| | **D10***^{a}* | **W2***^{b}* | **3D7***^{a}* | **K1***^{b}* | **NF54*^{a}*** | **KB cell***^{c}* | **NSO cells***^{d}* | **Daudi cells***^{e}* | **Normal human lymphocytes***^{f}* |
| **9a** | 20.7 | 22.4 | 24.5 | 65 | 162 | n.t. | 23.81 | 34.63 | 54.11 |
| **9b** | 141.9 | 219.6 | n.t.*^{g}* | 259.2 | 154.7 | n.t. | 44.73 | 43.90 | 40.76 |
| **9c** | n.t. | n.t. | n.t. | 19 | 13 | n.t. | n.t. | n.t. | n.t. |
| **9d** | n.t. | n.t. | n.t. | 107 | 47 | n.t. | n.t. | n.t. | n.t. |
| **9e** | 45.78 | 72.12 | 3.7 | 9 | 15 | 19.61 | 8.65 | 8.65 | 17.30 |
| **9f** | n.t. | n.t. | n.t. | 504. | 337 | n.t. | n.t. | n.t. | n.t. |
| **9g** | n.t. | n.t. | n.t. | 22 | 17 | n.t. | 1.68 | 1.47 | 1.42 |
| **9h** | n.t. | n.t. | n.t. | 20 | 13 | n.t. | n.t. | n.t. | n.t. |
| **9j** | n.t. | n.t. | n.t. | 375 | 163 | n.t. | n.t. | n.t. | n.t. |
| **9k** | 61.48 | 90.47 | 0.2 | 30 | 20 | 5.49 | 12.10 | 14.11 | 24.20 |
| **9l** | n.t. | n.t. | n.t. | 33 | 19.8 | n.t. | n.t. | n.t. | n.t. |
| **9m** | n.t. | n.t. | n.t. | 65.5 | 39.3 | n.t. | n.t. | n.t. | n.t. |
| **9n** | n.t. | n.t. | n.t. | 67 | 43 | n.t. | n.t. | n.t. | n.t. |
| **9o** | n.t. | n.t. | n.t. | 34 | 27 | n.t. | n.t. | n.t. | n.t. |
| **9p** | 57.18 | 81.90 | 8 | 60 | 32 | 8.5 | n.t. | n.t. | n.t. |
| **9q** | n.t. | n.t. | n.t. | 54 | 33 | n.t. | n.t. | n.t. | n.t. |
| **9r** | n.t. | n.t. | n.t. | 58 | 21 | n.t. | n.t. | n.t. | n.t. |
| **9s** | 48.65 | 58.76 | 1 | 8 | 167 | n.t. | 11.06 | 11.06 | 16.59 |
| **15a** | n.t. | n.t. | 3 | 22 | n.t. | n.t. | 14.86 | 16.71 | 18.57 |
| **CLT** | 550 | 490 | 60 | 250 | n.t. | 88.1 | n.t. | n.t. | n.t. |
| **CQ** | 22 | 280 | 10 | 260 | 7.0 | 207.0 | n.t. | n.t. | n.t. |

| | | | | | | | | | |
|---|---|---|---|---|---|---|---|---|---|
| *^{a}*CQ-S clone. *^{b}*CQ-R clone. *^{c}*Human carcinoma of the nasopharynx cell line. *^{d}*Plasmocytoma murine cell line. *^{e}*Human lymphoblastoid cell line. *^{f}*Normal human lymphocytes PHA-stimulated. *^{g}*n.t. = not tested. | | | | | | | | | |

### Example 4

### In vivo activity of 9a,e,k,s and 15a

Compounds **9a,e,k,s and 15a** were tested *in vivo* against *P. chabaudi* and CLT and CQ were used as reference drugs. The experiment was performed at a single dose of 50 mg/Kg. ED₅₀ was determined for **9a** in *P. berghei ANKA*. The results are shown in Table 3. All compounds were admistered per os.

**Table 3**

| **Compound** | ***P. Chabaudi AS*** **(% inhibition at 50 mg/Kg)** | ***P. Berghei ANKA*** **(ED₅₀, mg/Kg)** |
|---|---|---|
| **9a** | 98.0 | 6.3^{a} |
| **9e** | 99.5 | n.t. |
| **9k** | 99.6 | n.t. |
| **9s** | 99.9 | n.t. |
| **15a** | 99.9 | n.t. |
| **CLT** | 6.0 | - |
| **CQ** | 100 (10 mg/Kg) | - |

| | | |
|---|---|---|
| *^{a}*98% inhibition of parasitemia at 50 mg/Kg per os in *P. berghei.* | | |

### Example 5

### Antifungal activity of 9a,e,k,p

We tested yeast and filamentous fungi from international collection (*Candida krusei* ATCC® 6258, *Candida parapsilosis* ATCC® 22019 [both quality control strains], *Aspergillus flavus* ATCC^{®} 204304 [reference strain]) and the following clinical isolates (*Candida albicans* MGMI1, *Candida glabrata* MGMI2, *Cryptococcus neoformans* MGMI3 and two *Aspergillus fumigatus* MGMI4 e MGMI5, one (MGMI5) itraconazole (ITRA) resistant (MIC > 16 mcg/ml)) against compounds **7a-d** using the CLSI protocols for yeast and filamentous fungi described in
(1) National Committee for Clinical Laboratory Standards. (2002). Reference method for broth dilution antifungal susceptibility testing of yeasts - Second Edition: Approved Standard, M27-A2. NCCLS, Wayne, PA; and
(2) National Committee for Clinical Laboratory Standards. Reference method for broth dilution antifungal susceptibility testing of filamentous fungi: Approved Standard, M38-A. 2002; NCCLS, Wayne, PA.

The results are shown in Table 4.

**Table 4**

| **Strains** | **9a** | **9e** | **9k** | **9p** | **ITRA** | **CL T** |
|---|---|---|---|---|---|---|
| *Candida albicans* MGMI 1 | >32 | >32 | 16 | >32 | 0.25 | 0.03 |
| *Candida glabrata* MGMI 2 | >32 | >32 | >32 | >32 | 32 | NT |
| *Candida krusei* ATCC® 6258 | >32 | >32 | >32 | >32 | 0.25 | 2 |
| *Candida parapsilosis* ATCC® 22019 | 32 | 16 | 16 | >32 | 0.125 | 2 |
| *Cryptococcus neoformans* MGMI 3 | 16 | 16 | 16 | >32 | 0.25 | 2 |
| *Aspergillus flavus* ATCC^{®} 204304 | >32 | >32 | >32 | >32 | 0.06 | 4 |
| *Aspergillus fumigatus* MGMI 4 | >32 | >32 | >32 | >32 | 0.06 | 4 |
| *Aspergillus fumigatus* MGMI 5 | >32 | >32 | >32 | >32 | >32 | 4 |

The lack of anti-fungal activity of **9a,e,k,p** demonstrated that they selectively interact with free heme and not with heme as a prosthetic group in cythocromes. In fact, inhibition of cythocromes P450 through interaction with the heme prosthetic group is the mechanism of action of antifungal activity of CLT and related azoles.

### Example 6

### β-Hematin inhibitory activity

The β-hematin inhibitory activity of compounds **9a,** CLT (**2**) and CQ (**1**) were determined as described by Parapini et Al (Exp. Parasitol. 2000, 96, 249) by using the BHIA (β-hematin inhibitory activity) assay (hemin in dimethyl sulfoxide-acetate buffer at pH 5.0, 37°C, 18 h) and are presented in Table 5, below.

**Table 5**

| **Compound** | **IC₅₀*** |
|---|---|
| **9a** | 0.785 |
| **CLT** | 2.50 |
| **CQ** | 0.91±0.23 |
| **Amodiaquine** | 0.79±0.01 |

| | |
|---|---|
| * Molar equivalents of compound relative to hemin. | |

IC₅₀ are the mean of at least three determinations. Standard errors were all within 10% of the mean.

## Claims

1. A 4-amino-quinoline derivative represented by Formula I, 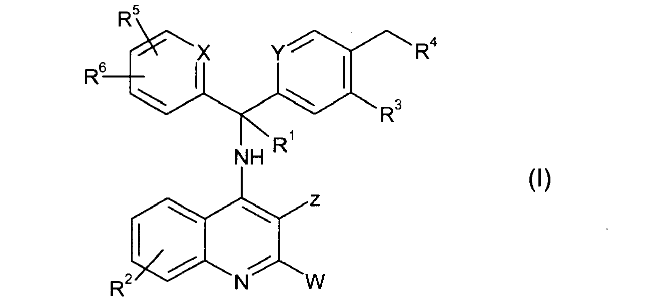 an isomer thereof or a mixture of its isomers, or a pharmaceutically acceptable salt thereof, wherein
X and Y both represent CH; or
X and Y, together with the carbon atoms to which they are attached, form a bridge selected from C-C, C-CH₂-CH₂-C and C-CH=CH-C;
W and Z both represent hydrogen; or
W and Z, together with the carbon atoms to which they are attached, form a benzo-fused ring -CH=CH-CH=CH-, which fused ring may optionally be substituted with halo;
R¹ represents hydrogen, phenyl, halo-substituted phenyl, 3,4-methylendioxyphenyl or (pyrrolidinylmethyl)phenyl;
R² represents halo, trifluoromethyl or alkoxy;
R³ represents hydrogen, halo, hydroxy, cyano, sulfonamido or dialkylsulfonamido;
R⁴ represents *N*,*N*-dialkyl-amino, pyrrolidinyl, piperazinyl, morpholinyl or imidazolyl;
R⁵ represents hydrogen, halo, cyano, hydroxy or -SO₂NH₂; and
R⁶ represents hydrogen, *N*,*N*-dialkyl-amino-methyl, pyrrolidinylmethyl, piperazinyl-methyl, morpholinyl-methyl or 1*H*-imidazolylmethyl.

2. The 4-amino-quinoline derivative of claim 1, an isomer thereof or a mixture of its isomers, or a pharmaceutically acceptable salt thereof, wherein X and Y both represent CH.

3. The 4-amino-quinoline derivative of claim 1, an isomer thereof or a mixture of its isomers, or a pharmaceutically acceptable salt thereof, wherein X and Y, together with the carbon atoms to which they are attached, form a bridge selected from C-C, C-CH₂-CH₂-C and C-CH=CH-C.

4. The 4-amino-quinoline derivative of claim 1, an isomer thereof or a mixture of its isomers, or a pharmaceutically acceptable salt thereof, wherein W and Z both represent hydrogen.

5. The 4-amino-quinoline derivative of claim 1, an isomer thereof or a mixture of its isomers, or a pharmaceutically acceptable salt thereof, wherein W and Z, together with the carbon atoms to which they are attached, form a benzo-fused ring -CH=CH-CH=CH-, which fused ring may optionally be substituted with halo.

6. The 4-amino-quinoline derivative of claim 1, an isomer thereof or a mixture of its isomers, or a pharmaceutically acceptable-salt thereof, wherein W and Z, together with the carbon atoms to which they are attached, form a benzo-fused ring selected from -CH=CH-CH=CH- and -CH=CH-C(Cl)=CH-.

7. The 4-amino-quinoline derivative of claim 1, an isomer thereof or a mixture of its isomers, or a pharmaceutically acceptable salt thereof, wherein
X and Y both represent CH;
W and Z both represent hydrogen;
R₁ represents hydrogen, phenyl, fluorophenyl, 3,4-methylendioxyphenyl or (pyrrolidinylmethyl)phenyl;
R₂ represents halo, trifluoromethyl or alkoxy;
R₃ represents hydrogen, halo, hydroxy, cyano, sulfonamido or dialkylsulfonamido;
R₄ represents *N*,*N*-dialkyl-amino, pyrrolidinyl, piperazynyl, morpholinyl or imidazolyl;
R₅ represents hydrogen, halo, cyano, hydroxy or SO₂NH₂; and
R₆ represents hydrogen, *N*,*N*-dialkyl-aminomethyl, pyrrolidinylmethyl, piperazynylmethyl, morpholinylmethyl or imidazolylmethyl.

8. The 4-amino-quinoline derivative of claim 1, an isomer thereof or a mixture of its isomers, or a pharmaceutically acceptable salt thereof, wherein
X and Y both represent CH;
W and Z, together with the carbon atoms to which they are attached, form a benzo-fused ring -CH=CH-CH=CH-, which fused ring may optionally be substituted with halo;
R₁ represents hydrogen, phenyl, flurophenyl, 3,4-methylendioxyphenyl or (pyrrolidinylmethyl)phenyl;
R₂ represents alkoxy;
R₃ represents hydrogen, halo, hydroxy, cyano, sulfonamido or dialkylsulfonamido;
R₄ represents *N,N*-dialkyl-amino, pyrrolidinyl, piperazynyl, morpholinyl or imidazolyl;
R₅ represents hydrogen, halo, cyano, hydroxy or SO₂NH₂; and
R₆ represents hydrogen, *N*,*N*-dialkyl-aminomethyl, pyrrolidinylmethyl, piperazynylmethyl, morpholinylmethyl or imidazolylmethyl.

9. The 4-amino-quinoline derivative of claim 1, an isomer thereof or a mixture of its isomers, or a pharmaceutically acceptable salt thereof, wherein
X and Y, together with the carbon atoms to which they are attached, form a bridge selected from C-C, C-CH₂-CH₂-C and C-CH=CH-C; and
W and Z both represent hydrogen; or
W and Z, together with the carbon atoms to which they are attached, form a benzo-fused ring -CH=CH-CH=CH-, which fused ring may optionally be substituted with halo;
R₁ represents hydrogen;
R₂ represents halo, trifluoromethyl or alkoxy;
R₃ represents hydrogen, halo, hydroxy, cyano, sulfonamido or dialkylsulfonamido;
R₄ represents *N*,*N*-dialkyl-amino, pyrrolidinyl, piperazynyl, morpholinyl or imidazolyl;
R₅ represents hydrogen, halo or hydroxy; and
R₆ represents hydrogen, *N*,*N*-dialkyl-aminomethyl, pyrrolidinylmethyl, piperazynylmethyl, morpholinylmethyl or imidazolylmethyl.

10. The 4-amino-quinoline derivative of claim 1, which is
(±)-7-Chloro-*N*-{(3-chlorophenyl)[4-(pyrrolidin-1-ylmethyl)phenyl]methyl}-4-aminoquinoline;
(±)-7-Chloro-*N*-{(3-chlorophenyl)[4-(morpholin-4-ylmethyl)phenyl]methyl}-4-aminoquinoline;
(±)-7-Chloro-*N*-{[3-chloro-4-(pyrrolidin-1-ylmethyl)phenyl](3-chlorophenyl)methyl}-4-aminoquinoline;
(±)-7-Chloro-*N*-{[3-chloro-4-(morpholin-4-ylmethyl)phenyl](3-chlorophenyl)methyl}-4-aminoquinoline;
(±)-7-Chloro-*N*-{(4-chlorophenyl)[4-(pyrrolidin-1-ylmethyl)phenyl]methyl}-4-aminoquinoline;
(±)-7-Chloro-*N*-{(4-chlorophenyl)[4-(morpholin-4-ylmethyl)phenyl]methyl}-4-aminoquinoline;
(±)-7-Chloro-*N*-{(4-chlorophenyl)[4-(piperazin-1-ylmethyl)phenyl]methyl}-4-aminoquinoline;
(±)-7-Chloro-*N*-{(4-chlorophenyl)[3-fluoro-4-(pyrrolidin-1-ylmethyl)phenyl]methyl}-4-aminoquinoline;
(±)-*N*-{[3-Chloro-4-(pyrrolidin-1-ylmethyl)phenyl](3-chlorophenyl)methyl}-7-trifluoromethyl-4-aminoquinoline;
(±)-*N*-{[3-Chloro-4-(morpholin-4-ylmethyl)phenyl](3-chlorophenyl)methyl}-7-trifluoromethyl-4-aminoquinoline;
(±)-*N*-{(4-Chlorophenyl)[4-(pyrrolidin-1-ylmethyl)phenyl]methyl}-7-trifluoromethyl-4-aminoquinoline;
(±)-*N*-{(4-Chlorophenyl)[3-fluoro-4-(pyrrolidin-1-ylmethyl)phenyl]methyl}-7-trifluoromethyl-4-aminoquinoline;
(±)-6-Methoxy-*N*-{(3-chlorophenyl)[4-(pyrrolidin-1-ylmethyl)phenyl]methyl}-4-aminoquinoline;
(±)-*N*-{(3-Chlorophenyl)[4-(morpholin-4-ylmethyl)phenyl]methyl}-6-methoxy-4-aminoquinoline;
(±)-*N*-{[3-Chloro-4-(pyrrolidin-1-ylmethyl)phenyl](3-chlorophenyl)methyl}-6-methoxy-4-aminoquinoline;
(±)-6-Methoxy-*N*-{(4-chlorophenyl)[4-(pyrrolidin-1-ylmethyl)phenyl]methyl}-4-aminoquinoline;
(±)-*N*-{(4-Chlorophenyl)[4-(morpholin-4-ylmethyl)phenyl]methyl}-6-methoxy-4-aminoquinoline;
(±)-*N*-((4-Chlorophenyl)[4-(piperazin-1-ylmethyl)phenyl]methyl)-6-methoxy-4-aminoquinotine;
(±)-6-Chloro-*N-*{(4-chlorophenyl)[4-(pyrrolidin-1-ylmethyl)phenyl]methyl}-2-methoxy-9-aminoacridine;
(±)-7-Chloro-*N*-{[4-(1*H*-imidazol-1-yl)methylphenyl](4-chlorophenyl)methyl}-4-aminoquinoline;
(±)-7-Chloro-*N*-{(4-chlorophenyl)[4-(pyrrolidin-1-ylmethyl)phenyl]phenyl methyl}-4-aminoquinoline;
(±)-7-Chloro-*N*-{(4-chlorophenyl)(4-fluorophenyl)[4-(pyrrolidin-1-ylmethyl)phenyl]methyl}-4-aminoquinoline;
(±)-*N*-{(4-Chlorophenyl)[4-(pyrrolidin-1-ylmethyl)phenyl]phenylmethyl}-6-chloro-2-methoxy-9-aminoacridine; or
*N*-{bis[3-Chloro-4-(pyrrolidin-1-ylmethyl)phenyl]phenylmethyl}-7-chloro-4-aminoquinoline;
or a pharmaceutically acceptable addition salt thereof.

11. A pharmaceutical composition comprising a therapeutically effective amount of a 4-amino-quinoline derivative of any one of claims 1-10, or a pharmaceutically-acceptable addition salt thereof, together with one or more adjuvants, excipients, carriers and/or diluents.

12. Use of a 4-amino-quinoline derivative of any one of claims 1-10, or a pharmaceutically-acceptable addition salt thereof, for the manufacture of a pharmaceutical composition/medicament.

13. Use of a 4-amino-quinoline derivative of any one of claims 1-10, or a pharmaceutically-acceptable addition salt thereof, for the manufacture of a pharmaceutical composition/medicament for the treatment, prevention or alleviation of an infectious disease, disorder or condition of a mammal, including a human, which disease, disorder or condition is caused by a parasite of the genus *Plasmodium.*

14. The use according to claim 13, wherein the disease, disorder or condition is caused by *P. falciparum, P. berghei, P. vivax, P. ovale, P. malaria* or *P. knowlesi.*

15. The use according to claim 13, wherein the disease, disorder or condition is malaria.

16. The 4-amino-quinoline derivative of any one of claims 1-10, or a pharmaceutically-acceptable addition salt thereof, for use as a medicament.

## Patentansprüche

1. 4-Amino-chinolin-Derivat, das durch Formel I wiedergegeben ist 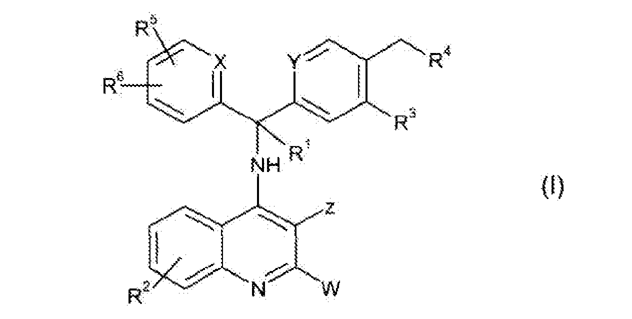 ein Isomer davon oder eine Mischung von seinen Isomeren, oder ein pharmazeutisch verträgliches Salz davon, worin
X und Y beide CH bedeuten; oder
X und Y, zusammen mit den Kohlenstoffatomen, an die sie gebunden sind, eine Brücke bilden, die ausgewählt ist aus C-C, C-CH₂-CH₂-C und C-CH=CH-C;
W und Z beide Wasserstoff bedeuten; oder
W und Z, zusammen mit den Kohlenstoffatomen, an die sie gebunden sind, einen Benzo-kondensierten Ring -CH=CH-CH=CH- bilden, wobei dieser kondensierte Ring gegebenenfalls mit Halogen substituiert sein kann;
R¹ Wasserstoff, Phenyl, halogensubstituiertes Phenyl, 3,4-Methylendioxyphenyl oder (Pyrrolidinylmethyl)phenyl bedeutet;
R² Halogen, Trifluormethyl oder Alkoxy bedeutet;
R³ Wasserstoff, Halogen, Hydroxy, Cyano, Sulfonamido oder Dialkylsulfonamido bedeutet;
R⁴ *N*,*N*-Dialkyl-amino, Pyrrolidinyl, Piperazinyl, Morpholinyl oder Imidazolyl bedeutet;
R⁵ Wasserstoff, Halogen, Cyano, Hydroxy oder -SO₂NH₂ bedeutet; und
R⁶ Wasserstoff, *N*,*N*-Dialkyl-amino-methyl, Pyrrolidinyl-methyl, Piperazinyl-methyl, Morpholinyl-methyl oder 1*H*-Imidazolyl-methyl bedeutet.

2. 4-Amino-chinolin-Derivat nach Anspruch 1, ein Isomer davon oder eine Mischung von seinen Isomeren, oder ein pharmazeutisch verträgliches Salz davon, worin X und Y beide CH bedeuten.

3. 4-Amino-chinolin-Derivat nach Anspruch 1, ein Isomer davon oder eine Mischung von seinen Isomeren, oder ein pharmazeutisch verträgliches Salz davon, worin X und Y, zusammen mit den Kohlenstoffatomen, an die sie gebunden sind, eine Brücke bilden, die ausgewählt ist aus C-C, C-CH₂-CH₂-C und C-CH=CH-C.

4. 4-Amino-chinolin-Derivat nach Anspruch 1, ein Isomer davon oder eine Mischung von seinen Isomeren, oder ein pharmazeutisch verträgliches Salz davon, worin W und Z beide Wasserstoff bedeuten.

5. 4-Amino-chinolin-Derivat nach Anspruch 1, ein Isomer davon oder eine Mischung von seinen Isomeren, oder ein pharmazeutisch verträgliches Salz davon, worin W und Z, zusammen mit den Kohlenstoffatomen, an die sie gebunden sind, einen Benzo-kondensierten Ring -CH=CH-CH=CH- bilden, wobei dieser kondensierte Ring gegebenenfalls mit Halogen substituiert sein kann.

6. 4-Amino-chinolin-Derivat nach Anspruch 1, ein Isomer davon oder eine Mischung von seinen Isomeren, oder ein pharmazeutisch verträgliches Salz davon, worin W und Z, zusammen mit den Kohlenstoffatomen, an die sie gebunden sind, einen Benzo-kondensierten Ring bilden, der ausgewählt ist aus -CH=CH-CH=CH- und -CH=CH-C(CI)=CH-.

7. 4-Amino-chinolin-Derivat nach Anspruch 1, ein Isomer davon oder eine Mischung von seinen Isomeren, oder ein pharmazeutisch verträgliches Salz davon, worin
X und Y beide CH bedeuten;
W und Z beide Wasserstoff bedeuten;
R¹ Wasserstoff, Phenyl, Fluorphenyl, 3,4-Methylendioxyphenyl oder (Pyrrolidinylmethyl)phenyl bedeutet;
R² Halogen, Trifluormethyl oder Alkoxy bedeutet;
R³ Wasserstoff, Halogen, Hydroxy, Cyano, Sulfonamido oder Dialkylsulfonamido bedeutet;
R⁴ *N*,*N*-Dialkyl-amino, Pyrrolidinyl, Piperazinyl, Morpholinyl oder Imidazolyl bedeutet;
R⁵ Wasserstoff, Halogen, Cyano, Hydroxy oder SO₂NH₂ bedeutet; und
R⁶ Wasserstoff, *N*,*N*-Dialkyl-aminomethyl, Pyrrolidinylmethyl, Piperazinylmethyl, Morpholinylmethyl oder Imidazolylmethyl bedeutet.

8. 4-Amino-chinolin-Derivat nach Anspruch 1, ein Isomer davon oder eine Mischung von seinen Isomeren, oder ein pharmazeutisch verträgliches Salz davon, worin
X und Y beide CH bedeuten;
W und Z, zusammen mit den Kohlenstoffatomen, an die sie gebunden sind, einen Benzo-kondensierten Ring -CH=CH-CH=CH- bilden, wobei dieser kondensierte Ring gegebenenfalls mit Halogen substituiert sein kann;
R¹ Wasserstoff, Phenyl, Fluorphenyl, 3,4-Methylendioxyphenyl oder (Pyrrolidinylmethyl)phenyl bedeutet;
R² Alkoxy bedeutet;
R³ Wasserstoff, Halogen, Hydroxy, Cyano, Sulfonamido oder Dialkylsulfonamido bedeutet;
R⁴ *N*,*N*-Dialkyl-amino, Pyrrolidinyl, Piperazinyl, Morpholinyl oder Imidazolyl bedeutet;
R⁵ Wasserstoff, Halogen, Cyano, Hydroxy oder SO₂NH₂ bedeutet; und
R⁶ Wasserstoff, *N*,*N*-Dialkyl-aminomethyl, Pyrrolidinylmethyl, Piperazinylmethyl, Morpholinylmethyl oder Imidazolylmethyl bedeutet.

9. 4-Amino-chinolin-Derivat nach Anspruch 1, ein Isomer davon oder eine Mischung von seinen Isomeren, oder ein pharmazeutisch verträgliches Salz davon, worin
X und Y, zusammen mit den Kohlenstoffatomen, an die sie gebunden sind, eine Brücke bilden, die ausgewählt ist aus C-C, C-CH₂-CH₂-C und C-CH=CH-C; und
W und Z beide Wasserstoff bedeuten; oder
W und Z, zusammen mit den Kohlenstoffatomen, an die sie gebunden sind, einen Benzo-kondensierten Ring -CH=CH-CH=CH- bilden, wobei dieser kondensierte Ring gegebenenfalls mit Halogen substituiert sein kann;
R¹ Wasserstoff bedeutet;
R² Halogen, Trifluormethyl oder Alkoxy bedeutet;
R³ Wasserstoff, Halogen, Hydroxy, Cyano, Sulfonamido oder Dialkylsulfonamido bedeutet;
R⁴ *N*,*N*-Dialkyl-amino, Pyrrolidinyl, Piperazinyl, Morpholinyl oder Imidazolyl bedeutet;
R⁵ Wasserstoff, Halogen oder Hydroxy bedeutet; und
R⁶ Wasserstoff, *N*,*N*-Dialkyl-aminomethyl, Pyrrolidinylmethyl, Piperazinylmethyl, Morpholinylmethyl oder Imidazolylmethyl bedeutet.

10. 4-Amino-chinolin-Derivat nach Anspruch 1, welches
(±)-7-Chlor-*N*-{(3-chlorphenyl)[4-(pyrrolidin-1-ylmethyl)phenyl]methyl}-4-aminochinolin;
(±)-7-Chlor-*N*-{(3-chlorphenyl)[4-(morpholin-4-ylmethyl)phenyl]methyl}-4-aminochinolin;
(±)-7-Chlor-*N*-{(3-chlor-4-(pyrrolidin-1-ylmethyl)phenyl](3-chlorphenyl)methyl}-4-aminochinolin;
(±)-7-Chlor-*N*-{(3-chlor-4-(morpholin-4-ylmethyl)phenyl](3-chlorphenyl)methyl}-4-aminochinolin;
(±)-7-Chlor-*N*-{(4-chlorphenyl)[4-(pyrrolidin-1-ylmethyl)phenyl]methyl}-4-aminochinolin;
(±)-7-Chlor-*N*-{(4-chlorphenyl)[4-(morpholin-4-ylmethyl)phenyl]methyl}-4-aminochinolin;
(±)-7-Chlor-*N*-{(4-chlorphenyl)[4-(piperazin-1-ylmethyl)phenyl]methyl}-4-aminochinolin;
(±)-7-Chlor-*N*-{(4-chlorphenyl)[3-fluor-4-(pyrrolidin-1-ylmethyl)phenyl]methyl}-4-aminochinolin;
(±)-*N*-{[3-Chlor-4-(pyrrolidin-1-ylmethyl)phenyl](3-chlorphenyl)methyl}-7-trifluormethyl-4-aminochinolin;
(±)-*N*-{[3-Chlor-4-(morpholin-4-ylmethyl)phenyl](3-chlorphenyl)methyl}-7-trifluormethyl-4-aminochinolin;
(±)-*N*-((4-Chlorphenyl)[4-(pyrrolidin-1-ylmethyl)phenyl]methyl)-7-trifluormethyl-4-aminochinolin;
(±)-*N*-{(4-Chlorphenyl)[3-fluor-4-(pyrrolidin-1-ylmethyl)phenyl]methyl}-7-trifluormethyl-4-aminochinolin;
(±)-6-Methoxy-*N*-{(3-chlorphenyl)[4-(pyrrolidin-1-ylmethyl)phenyl]methyl}-4-aminochinolin;
(±)-*N*-{(3-Chlorphenyl)[4-(morpholin-4-ylmethyl)phenyl]methyl}-6-methoxy-4-aminochinolin;
(±)-*N*-{[3-Chlor-4-(pyrrolidin-1-ylmethyl)phenyl](3-chlorphenyl)methyl}-6-methoxy-4-aminochinolin;
(±)-6-Methoxy-*N*-{(4-chlorphenyl)[4-(pyrrolidin-1-ylmethyl)phenyl]methyl}-4-aminochinolin;
(±)-*N*-{(4-Chlorphenyl)[4-(morpholin-4-ylmethyl)phenyl]methyl}-6-methoxy-4-aminochinolin;
(±)-*N*-{(4-Chlorphenyl)[4-(piperazin-1-ylmethyl)phenyl]methyl}-6-methoxy-4-aminochinolin;
(±)-6-Chlor-*N*-{(4-chlorphenyl)[4-(pyrrolidin-1-ylmethyl)phenyl]methyl}-2-methoxy-9-aminoacridin;
(±)-7-Chlor-*N*-{[4-(1*H*-imidazol-1-yl)methylphenyl](4-chlorphenyl)methyl}-4-aminochinolin;
(±)-7-Chlor-*N*-{(4-chlorphenyl)[4-(pyrrolidin-1-ylmethyl)phenyl]phenylmethyl}-4-aminochinolin;
(±)-7-Chlor-*N*-{(4-chlorphenyl)(4-fluorphenyl)[4-(pyrrolidin-1-ylmethyl)phenyl]methyl}-4-aminochinolin;
(±)-*N*-{(4-Chlorphenyl)[4-(pyrrolidin-1-ylmethyl)phenyl]phenylmethyl}-6-chlor-2-methoxy-9-aminoacridin; oder
*N*-{bis[3-Chlor-4-(pyrrolidin-1-ylmethyl)phenyl]phenylmethyl)-7-chlor-4-aminochinolin ist;
oder ein pharmazeutisch verträgliches Additionssalz davon.

11. Pharmazeutische Zusammensetzung, umfassend eine therapeutisch wirksame Menge eines 4-Amino-chinolin-Derivats nach einem der Ansprüche 1-10, oder eines pharmazeutisch verträglichen Additionssalzes davon, zusammen mit einem oder mehreren Adjuvanzien, Exzipienten, Trägern und/oder Verdünnungsmitteln.

12. Verwendung eines 4-Amino-chinolin-Derivats nach einem der Ansprüche 1-10, oder eines pharmazeutisch verträglichen Additionssalzes davon, für die Herstellung einer pharmazeutischen Zusammensetzung/eines Medikaments.

13. Verwendung eines 4-Amino-chinolin-Derivats nach einem der Ansprüche 1-10, oder eines pharmazeutisch verträglichen Additionssalzes davon, für die Herstellung einer pharmazeutischen Zusammensetzung/eines Medikaments für die Behandlung, Verhütung oder Linderung einer infektiösen Krankheit, einer Störung oder eines Zustands eines Säugers, einschließlich eines Menschen, wobei diese Krankheit, diese Störung oder dieser Zustand durch einen Parasiten der Gattung *Plasmodium* verursacht wird.

14. Verwendung nach Anspruch 13, wobei die Krankheit, die Störung oder der Zustand durch *P. falciparum, P. berghei, P. vivax, P. ovale, P. malaria* oder *P. knowlesi* verursacht wird.

15. Verwendung nach Anspruch 13, wobei es sich bei der Krankheit, der Störung oder dem Zustand um Malaria handelt.

16. 4-Amino-chinolin-Derivat nach einem der Ansprüche 1-10, oder ein pharmazeutisch verträgliches Additionssalz davon, zur Verwendung als ein Medikament.

## Revendications

1. Dérivé de 4-amino-quinoléine représenté par la formule I, 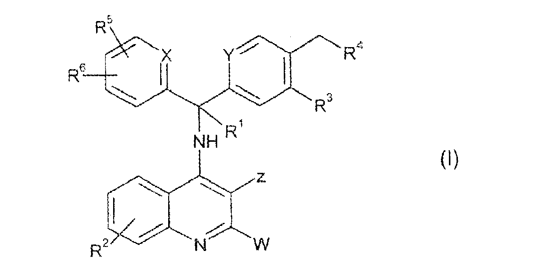 un isomère de celui-ci ou un mélange de ses isomères, ou un sel pharmaceutiquement acceptable de celui-ci, dans lequel
X et Y représentent tous les deux CH ; ou
X et Y, conjointement avec les atomes de carbone auxquels ils sont attachés, forment un pont choisi parmi C-C, C-CH₂-CH₂-C et C-CH=CH-C ;
W et Z représentent tous les deux un hydrogène ; ou
W et Z, conjointement avec les atomes de carbone auxquels ils sont attachés, forment un cycle benzo-condensé -CH=CH-CH=CH-, lequel cycle condensé peut être éventuellement substitué par un halogéno ;
R¹ représente un hydrogène, un phényle, un phényle substitué par un halogéno, un 3,4-méthylènedioxyphényle ou un (pyrrolidinylméthyl)phényle ;
R² représente un halogéno, un trifluorométhyle ou un alcoxy ;
R³ représente un hydrogène, un halogéno, un hydroxy, un cyano, un sulfonamido ou un dialkylsulfonamido ;
R⁴ représente un *N*,*N*-dialkylamino, un pyrrolidinyle, un pipérazinyle, un morpholinyle ou un imidazolyle ;
R⁵ représente un hydrogène, un halogéno, un cyano, un hydroxy ou -SO₂NH₂ ; et
R⁶ représente un hydrogène, un *N*,*N*-dialkylaminométhyle, un pyrrolidinylméthyle, un pipérazinylméthyle, un morpholinylméthyle ou un 1*H*-imidazolylméthyle.

2. Dérivé de 4-aminoquinoléine selon la revendication 1, un isomère de celui-ci ou un mélange de ses isomères, ou un sel pharmaceutiquement acceptable de celui-ci, dans lequel X et Y représentent tous les deux CH.

3. Dérivé de 4-aminoquinoléine selon la revendication 1, un isomère de celui-ci ou un mélange de ses isomères, ou un sel pharmaceutiquement acceptable de celui-ci, dans lequel X et Y, conjointement avec les atomes de carbone auxquels ils sont attachés, forment un pont choisi parmi C-C, C-CH₂-CH₂-C et C-CH=CH-C.

4. Dérivé de 4-aminoquinoléine selon la revendication 1, un isomère de celui-ci ou un mélange de ses isomères, ou un sel pharmaceutiquement acceptable de celui-ci, dans lequel W et Z représentent tous les deux un hydrogène.

5. Dérivé de 4-aminoquinoléine selon la revendication 1, un isomère de celui-ci ou un mélange de ses isomères, ou un sel pharmaceutiquement acceptable de celui-ci, dans lequel W et Z, conjointement avec les atomes de carbone auxquels ils sont attachés, forment un cycle benzo-condensé -CH=CH-CH=CH-, lequel cycle condensé peut être éventuellement substitué par un halogéno.

6. Dérivé de 4-aminoquinoléine selon la revendication 1, un isomère de celui-ci ou un mélange de ses isomères, ou un sel pharmaceutiquement acceptable de celui-ci, dans lequel W et Z, conjointement avec les atomes de carbone auxquels ils sont attachés, forment un cycle benzo-condensé choisi parmi -CH=CH-CH=CH- et -CH=CH-C(Cl)=CH-.

7. Dérivé de 4-aminoquinoléine selon la revendication 1, un isomère de celui-ci ou un mélange de ses isomères, ou un sel pharmaceutiquement acceptable de celui-ci, dans lequel
X et Y représentent tous les deux CH ;
W et Z représentent tous les deux un hydrogène ;
R¹ représente un hydrogène, un phényle, un fluorophényle, un 3,4-méthylènedioxyphényle ou un (pyrrolidinylméthyl)phényle ;
R² représente un halogéno, un trifluorométhyle ou un alcoxy ;
R³ représente un hydrogène, un halogéno, un hydroxy, un cyano, un sulfonamido ou un dialkylsulfonamido ;
R⁴ représente un *N*,*N*-dialkylamino, un pyrrolidinyle, un pipérazinyle, un morpholinyle ou un imidazolyle ;
R⁵ représente un hydrogène, un halogéno, un cyano, un hydroxy ou -SO₂NH₂ ; et
R⁶ représente un hydrogène, un *N*,*N*-dialkylaminométhyle, un pyrrolidinylméthyle, un pipérazinylméthyle, un morpholinylméthyle ou un imidazolylméthyle.

8. Dérivé de 4-aminoquinoléine selon la revendication 1, un isomère de celui-ci ou un mélange de ses isomères, ou un sel pharmaceutiquement acceptable de celui-ci, dans lequel
X et Y représentent tous les deux CH ;
W et Z, conjointement avec les atomes de carbone auxquels ils sont attachés, forment un cycle benzo-condensé -CH=CH-CH=CH-, lequel cycle condensé peut être éventuellement substitué par un halogéno ;
R¹ représente un hydrogène, un phényle, un fluorophényle, un 3,4-méthylènedioxyphényle ou un (pyrrolidinylméthyl)phényle ;
R² représente un alcoxy ;
R³ représente un hydrogène, un halogéno, un hydroxy, un cyano, un sulfonamido ou un dialkylsulfonamido ;
R⁴ représente un *N*,*N*-dialkylamino, un pyrrolidinyle, un pipérazinyle, un morpholinyle ou un imidazolyle ;
R⁵ représente un hydrogène, un halogéno, un cyano, un hydroxy ou -SO₂NH₂ ; et R⁶ représente un hydrogène, un *N*,*N*-dialkylaminométhyle, un pyrrolidinylméthyle, un pipérazinylméthyle, un morpholinylméthyle ou un imidazolylméthyle.

9. Dérivé de 4-aminoquinoléine selon la revendication 1, un isomère de celui-ci ou un mélange de ses isomères, ou un sel pharmaceutiquement acceptable de celui-ci, dans lequel
X et Y, conjointement avec les atomes de carbone auxquels ils sont attachés, forment un pont choisi parmi C-C, C-CH₂-CH₂-C et C-CH=CH-C ; et
W et Z représentent tous les deux un hydrogène ; ou
W et Z, conjointement avec les atomes de carbone auxquels ils sont attachés, forment un cycle benzo-condensé -CH=CH-CH=CH-, lequel cycle condensé peut être éventuellement substitué par un halogéno ;
R¹ représente un hydrogène ;
R² représente un halogéno, un trifluorométhyle ou un alcoxy ;
R³ représente un hydrogène, un halogéno, un hydroxy, un cyano, un sulfonamido ou un dialkylsulfonamido ;
R⁴ représente un *N*,*N*-dialkylamino, un pyrrolidinyle, un pipérazinyle, un morpholinyle ou un imidazolyle ;
R⁵ représente un hydrogène, un halogéno ou un hydroxy ; et
R⁶ représente un hydrogène, un *N*,*N*-dialkylaminométhyle, un pyrrolidinylméthyle, un pipérazinylméthyle, un morpholinylméthyle ou un imidazolylméthyle.

10. Dérivé de 4-aminoquinoléine selon la revendication 1, qui est
la (±)-7-chloro-*N*-{(3-chlorophényl)[4-(pyrrolidin-1-ylméthyl)phényl]méthyl}-4-aminoquinoléine ;
la (±)-7-chloro-*N*-{(3-chlorophényl)[4-(morpholin-4-ylméthyl)phényl]méthyl}-4-aminoquinoléine ;
la (±)-7-chloro-*N*-{[3-chloro-4-(pyrrolidin-1-ylméthyl)phényl](3-chlorophényl)-méthyl}-4-aminoquinoléine ;
la (±)-7-chloro-*N*-{[3-chloro-4-(morpholin-4-ylméthyl)phényl](3-chlorophényl)méthyl}-4-aminoquinoléine ;
la (±)-7-chloro-*N*-((4-chlorophényl)[4-(pyrrolidin-1-ylméthyl)phényl]méthyl)-4-aminoquinoléine ;
la (±)-7-chloro-*N*-{(4-chlorophényl)[4-(morpholin-4-ylméthyl)phényl]méthyl}-4-aminoquinoléine ;
la (±)-7-chloro-*N*-((4-chlorophényl)[4-(pipérazin-1-ylméthyl)phényl]méthyl)-4-aminoquinoléine ;
la (±)-7-chloro-*N*-{(4-chlorophényl)[3-fluoro-4-(pyrrolidin-1-ylméthyl)phényl]-méthyl}-4-aminoquinoléine ;
la (±)-*N*-{[3-chloro-4-(pyrrolidin-1-ylméthyl)phényl](3-chlorophényl)méthyl}-7-trifluorométhyl-4-aminoquinoléine ;
la (±)-*N*-{[3-chloro-4-(morpholin-4-ylméthyl)phényl](3-chlorophényl)méthyl}-7-trifluorométhyl-4-aminoquinoléine ;
la (±)-*N*-{(4-chlorophényl)[4-(pyrrolidin-1-ylméthyl)phényl]méthyl}-7-trifluorométhyl-4-aminoquinoléine ;
la (±)-*N*-((4-chlorophényl)[3-fluoro-4-(pyrrolidin-1-ylméthyl)phényl]méthyl)-7-trifluorométhyl-4-aminoquinoléine ;
la (±)-6-méthoxy-*N*-{(3-chlorophényl)[4-(pyrrolidin-1-ylméthyl)phényl]-méthyl}-4-aminoquinoléine ;
la (±)-*N*-{(3-chlorophényl)[4-(morpholin-4-ylméthyl)phényl]méthyl}-6-méthoxy-4-aminoquinoléine ;
la (±)-*N*-{[3-chloro-4-(pyrrolidin-1-ylméthyl)phényl](3-chlorophényl)méthyl}-6-méthoxy-4-aminoquinoléine ;
la (±)-6-méthoxy-*N*-{(4-chlorophényl)[4-(pyrrolidin-1-ylméthyl)phényl]-méthyl}-4-aminoquinoléine ;
la (±)-*N*-{(4-chlorophényl)[4-(morpholin-4-ylméthyl)phényl]méthyl}-6-méthoxy-4-aminoquinoléine ;
la (±)-*N*-{(4-chlorophényl)[4-(pipérazin-1-ylméthyl)phényl]méthyl}-6-méthoxy-4-aminoquinoléine ;
la (±)-6-chloro-*N*-{(4-chlorophényl)[4-(pyrrolidin-1-ylméthyl)phényl]méthyl}-2-méthoxy-9-aminoacridine ;
la (±)-7-chloro-*N*-{[4-(*1H*-imidazol-1-yl)méthylphényl](4-chlorophényl)-méthyl}-4-aminoquinoléine ;
la (±)-7-chloro-*N*-{(4-chlorophényl)[4-(pyrrolidin-1-ylméthyl)phényl]phénylméthyl}-4-aminoquinoléine ;
la (±)-7-chloro-*N*-{(4-chlorophényl)(4-fluorophényl)[4-(pyrrolidin-1-ylméthyl)-phényl]méthyl}-4-aminoquinoléine ;
la (±)-*N*-{(4-chlorophényl)[4-(pyrrolidin-1-ylméthyl)phényl]phénylméthyl}-6-chloro-2-méthoxy-9-aminoacridine ; ou
la *N*-{bis[3-chloro-4-(pyrrolidin-1-ylméthyl)phényl]phénylméthyl}-7-chloro-4-aminoquinoléine ;
ou un sel d'addition pharmaceutiquement acceptable de celui-ci.

11. Composition pharmaceutique comprenant une quantité thérapeutiquement efficace d'un dérivé de 4-aminoquinoléine selon l'une quelconque des revendications 1 à 10, ou d'un sel d'addition pharmaceutiquement acceptable de celui-ci, conjointement avec un ou plusieurs adjuvants, excipients, supports et/ou diluants.

12. Utilisation d'un dérivé de 4-aminoquinoléine selon l'une quelconque des revendications 1 à 10, ou d'un sel d'addition pharmaceutiquement acceptable de celui-ci, pour la fabrication d'une composition pharmaceutique/médicament.

13. Utilisation d'un dérivé de 4-aminoquinoléine selon l'une quelconque des revendications 1 à 10, ou d'un sel d'addition pharmaceutiquement acceptable de celui-ci, pour la fabrication d'une composition pharmaceutique/médicament pour le traitement, la prévention ou l'atténuation d'une maladie infectieuse, d'un trouble ou d'une affection d'un mammifère, incluant un être humain, laquelle maladie, lequel trouble ou laquelle affection est provoqué(e) par un parasite du genre *Plasmodium*.

14. Utilisation selon la revendication 13, dans laquelle la maladie, le trouble ou l'affection est provoqué(e) par *P. falciparum, P. berghei, P. vivax, P. ovale, P. malaria* ou *P. knowlesi*.

15. Utilisation selon la revendication 13, dans laquelle la maladie, le trouble ou l'affection est le paludisme.

16. Dérivé de 4-aminoquinoléine selon l'une quelconque des revendications 1 à 10, ou un sel d'addition pharmaceutiquement acceptable de celui-ci, pour une utilisation en tant que médicament.
